(19) Europäisches Patentamt
European Patent Office
Office européen des brevets

(11) EP 3 831 780 B1

(12) **EUROPEAN PATENT SPECIFICATION**

(45) Date of publication and mention of the grant of the patent:
**03.05.2023 Bulletin 2023/18**

(21) Application number: **20831466.6**

(22) Date of filing: **26.06.2020**

(51) International Patent Classification (IPC):
***H01M 10/0569** (2010.01)* ***H01M 10/0567** (2010.01)*
***H01M 10/0525** (2010.01)* ***C07D 473/12** (2006.01)*
*H01M 4/58 (2010.01)* *H01M 4/587 (2010.01)*
*H01M 10/0568 (2010.01)*

(52) Cooperative Patent Classification (CPC):
**C07D 473/12; H01M 10/0525; H01M 10/0567; H01M 10/0569;** H01M 4/5825; H01M 4/587; H01M 10/0568; H01M 2300/0028; Y02E 60/10

(86) International application number:
**PCT/JP2020/025384**

(87) International publication number:
**WO 2020/262670 (30.12.2020 Gazette 2020/53)**

(54) **NON-AQUEOUS ELECTROLYTE SOLUTION AND NON-AQUEOUS SECONDARY BATTERY**

NICHTWÄSSRIGE ELEKTROLYTLÖSUNG UND NICHTWÄSSRIGE SEKUNDÄRBATTERIE

SOLUTION D'ÉLECTROLYTE NON-AQUEUSE ET BATTERIE SECONDAIRE NON-AQUEUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2019 JP 2019121648**
**10.06.2020 JP 2020100869**

(43) Date of publication of application:
**09.06.2021 Bulletin 2021/23**

(73) Proprietor: **Asahi Kasei Kabushiki Kaisha**
**Tokyo 1000006 (JP)**

(72) Inventors:
- **SHOJI, Kosuke**
  **Tokyo 100-0006 (JP)**
- **MATSUOKA, Naoki**
  **Tokyo 100-0006 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**EP-A1- 3 279 997** **WO-A1-2018/083937**
**JP-A- 2001 148 257** **JP-A- 2001 307 737**
**JP-A- 2014 154 247** **JP-A- 2017 010 924**
**JP-A- 2020 047 394** **US-A1- 2012 251 892**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 831 780 B1

## Description

FIELD

The present invention relates to a nonaqueous electrolyte solution and to a nonaqueous secondary battery.

BACKGROUND

Nonaqueous secondary batteries, such as lithium ion batteries (LIB), have lightweight, high-energy and long-lasting characteristics and are therefore widely used as power sources for a variety of portable electronic devices. In recent years, nonaqueous secondary batteries have also become common for industrial uses including power tools such as electric power tools, and for mounting in vehicles such as electric vehicles and electric bicycles, and are also of interest in the field of electric power storage, such as home power storage systems.

Lithium ion batteries usually employ nonaqueous electrolyte solutions. For example, it is common to use solvents that are combinations of high dielectric solvents such as cyclic carbonate esters and low-viscosity solvents such as lower linear carbonate esters. However, most high dielectric solvents not only have high melting points but can also lead to deterioration of the load characteristics (output characteristics) and low-temperature characteristics of nonaqueous secondary batteries.

Lead acid batteries are used as in-vehicle batteries that are power sources for engine start-up. A number of problems are associated with lead acid batteries, including high environmental load, heavy weight, poor charge performance and the need for periodic replacement due to degradation. Moreover, as automobiles become more highly electricalized and the high-power load on batteries increases, lead acid batteries have become increasingly unable to meet the demands for higher capacity, higher output, lighter weight and longer life required for batteries. Therefore, efforts have been actively underway in recent years to use lithium ion batteries as alternatives to lead acid batteries. With the burgeoning of industries dealing with large electricity storage, especially for electric vehicles, there is increasing demand for ever higher functionality of nonaqueous secondary batteries.

One group of electrolyte solvents for nonaqueous secondary batteries that has been proposed as a means of solving this problem are nitrile-based solvents (such as acetonitrile), which have an excellent balance between viscosity and relative permittivity. However, because acetonitrile has the disadvantage of undergoing electrochemical reductive decomposition at the negative electrode, it has not yet been possible to exhibit its performance in a practical manner. Several ameliorating strategies have been proposed against this problem.

The major strategies proposed to date can be classified into the following three groups.

(1) Methods of protecting the negative electrode with combinations of specific electrolyte salts and additives, to inhibit reductive decomposition of acetonitrile.
PTL 1, for example, reports an electrolyte solution having reduced reductive decomposition of an acetonitrile solvent, by combination of the acetonitrile with a specific electrolyte salt and an additive. Electrolyte solutions containing solvents that are acetonitrile diluted with propylene carbonate and ethylene carbonate have also been reported, as in PTL 2. It has been difficult, however, to inhibit reductive decomposition of acetonitrile-containing electrolyte solutions by simple dilution with ethylene carbonate and propylene carbonate.

(2) Methods of inhibiting reductive decomposition of acetonitrile using negative electrode active materials that store lithium ion, and have greater electropositive potential than the reduction potential of acetonitrile.

For example, PTL 3 reports that a battery without reductive decomposition of acetonitrile can be obtained by using a specific metal compound for the negative electrode. However, when the ameliorating strategy of PTL 3 is applied for purposes that prioritize the energy density of lithium ion batteries, this disadvantageously narrows the range of voltages that can be used.

(3) Methods of maintaining liquid state stability by dissolving high-concentration electrolyte salts in acetonitrile

PTL 4, for example, teaches that when using an electrolyte solution comprising lithium bis(trifluoromethanesulfonyl)imide represented by the formula $LiN(SO_2CF_3)_2$ dissolved in acetonitrile to a concentration of 4.2 mol/L, it is possible to insert and detach lithium in a reversible manner into a graphite electrode.

Other strategies for improving load characteristics or cycle characteristics without focusing on the electrolyte solvent have also been reported. PTLs 5 to 7, for example, report that adding a specific nitrogen-containing cyclic compound as an additive to the electrolyte solution can result in satisfactory battery performance.

PTL 8, in addition, reports that adding a specific condensation polycyclic heterocyclic ring compound to the

positive electrode material can provide a positive electrode material with excellent capacity and cycle characteristics, which can be used in a battery with high safety and productivity.

For lithium ion batteries to be used as substitutes for lead acid batteries, no nonaqueous secondary battery is yet known that provides a satisfactory high level for both output characteristics during engine start-up in low temperature environments and durability in high-temperature environments such as engine rooms. Furthermore, no practical example yet exists where this has been achieved with a combination using an acetonitrile-containing electrolyte solution with excellent viscosity and permittivity.

JP 2001 148257 A discloses an electrolytic solution for battery wherein an organic salt is dissolved in a solvent for a non-aqueous electrolytic solution of a battery which has a positive electrode having an occlusion and discharge capability of lithium ion and has a negative electrode comprising a lithium or lithium alloy, wherein the organic solvent contains a triazol system which forms a stabilized film on a surface of the negative electrode and restrains a concentration of the current of negative electrode and a sub-reaction of the electrolytic solution with lithium.

US 2012/251892 A1 (PTL 9) discloses an electrolyte for a lithium secondary battery including a lithium salt, a nonaqueous organic solvent, and an additive, in which the additive is composed of one or more compounds including a purinone or a purinone derivative.

[CITATION LIST]

[PATENT LITERATURE]

[PTL 1] International Patent Publication No. WO2013/062056
[PTL 2] Japanese Unexamined Patent Publication No. H04 (1992)-351860
[PTL 3] Japanese Unexamined Patent Publication No. 2009-21134
[PTL 4] International Patent Publication No. WO2013/146714
[PTL 51 International Patent Publication No. WO2016/159117
[PTL 6] International Patent Publication No. WO2013/183673
[PTL 7] International Patent Publication No. WO2016/068022
[PTL 8] Japanese Unexamined Patent Publication No. 2001-307737
[PTL 9] U.S. Patent Application Publication No. 2012/0251892

SUMMARY

[TECHNICAL PROBLEM]

In the technologies described in PTLs 1 to 4, the lithium ion batteries that use acetonitrile-containing electrolyte solutions have inferior high-temperature durability compared to existing lithium ion batteries that use carbonate solvent-containing electrolyte solutions.

Moreover, while a specific nitrogen-containing cyclic compound is added to the electrolyte solution to improve the load characteristic and cycle characteristic in the technologies described in PTLs 5 and 6, self-discharge under high temperature has been considerable and it has not been possible to avoid lowering the residual capacity of the battery.

The technology described in PTL 7 is superior as an electrolyte solution for a capacitor, but from the viewpoint of use as an electrolyte solution for a lithium ion battery, it is expected that practical performance would not be exhibited since electrochemical decomposition of acetonitrile at the negative electrode is not inhibited.

With the technology described in PTL 8, the proportion of the positive electrode active material is reduced compared to a positive electrode material containing no additive, and therefore it is necessary to either reduce the battery capacity or to increase the basis weight of the positive electrode. When additives elute into the electrolyte solution there is a potential for partial disintegration of the positive electrode structure, and therefore only limited types of additives can be used. Methods for adding additives to the positive electrode material are also limited to methods that do not cause degeneration of the additives. In PTL 8, the actual combination of the positive electrode material and additives is by physical mixing, with no other methods of addition being mentioned.

PTL 9 mentions an electrolyte solution containing a specific nitrogen-containing cyclic compound, but it does not deal with acetonitrile-containing electrolyte solutions.

Previously, the present inventors have discovered an acetonitrile electrolyte solution that exhibits excellent battery performance in storage testing at 85°C for 4 hours. However, the battery performance has been found to degrade with more prolonged storage testing. This is a recent finding by the present inventors that is not reflected in PTLs 1 to 8.

It is therefore an object of the present invention to provide, firstly, a nonaqueous electrolyte solution with reduced

degradation reaction in the nonaqueous secondary battery and reduced self-discharge at high temperatures above 60°C, as a result of reduced decomposition reaction of the nonaqueous electrolyte solution components by active oxygen species generated during the electrochemical reaction, and with improved output characteristics and cycle performance in a wide range of temperatures, as well as a nonaqueous secondary battery comprising the solution. It is another object of the invention to provide, secondly, a nonaqueous electrolyte solution wherein the mixing ratio of lithium (Li) salts is controlled to avoid lack of negative electrode SEI formation and corrosion of the aluminum (Al) current collector while also inhibiting degradation reaction in the nonaqueous secondary battery caused by heat degradation of Li salts, and which is able to improve the output characteristic of the nonaqueous secondary battery in a wide range of temperatures, as well as the long-term durability and cycle performance at high temperatures above 60°C, as well as a nonaqueous secondary battery comprising the solution.

[SOLUTION TO PROBLEM]

As a result of much diligent research, the present inventors have completed this invention upon finding that the problems described above can be solved by using a nonaqueous electrolyte solution or nonaqueous secondary battery having the following construction. The claimed invention is directed to the following:

[1] A nonaqueous electrolyte solution comprising:

a nonaqueous solvent containing acetonitrile at 5 vol% to 95 vol%;
a lithium salt; and
one or more compounds having a structure satisfying the following conditions 1 to 5:

1. being a condensation polycyclic heterocyclic ring compound,
2. containing a pyrimidine backbone in the condensation polycyclic heterocyclic ring,
3. containing 3 or more nitrogen atoms in the condensation polycyclic heterocyclic ring,
4. containing 5 or more sp2 carbons in the condensation polycyclic heterocyclic ring, and
5. having no hydrogen atoms bonded to the nitrogen atoms in the condensation polycyclic heterocyclic ring.

[2] The nonaqueous electrolyte solution according to [1] above, wherein the condensation polycyclic heterocyclic ring compound is at least one selected from the group consisting of compounds represented by the following formulas (1) to (12):

[Chemical Formula 1]

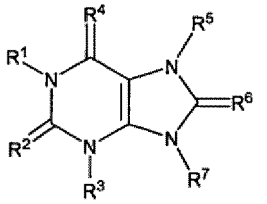 (1) 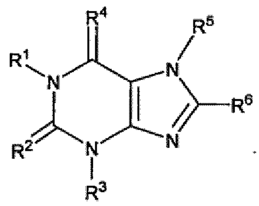 (2) 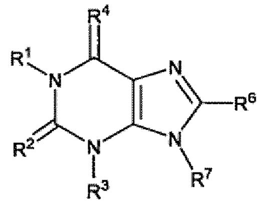 (3)

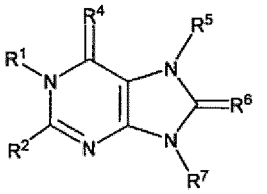 (4) 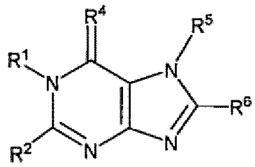 (5) 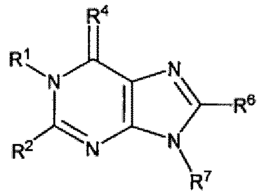 (6)

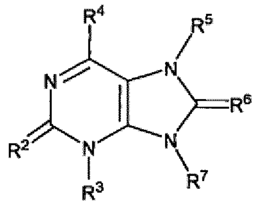 (7) 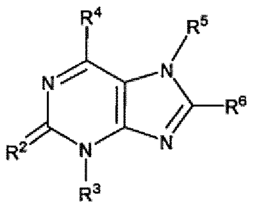 (8) 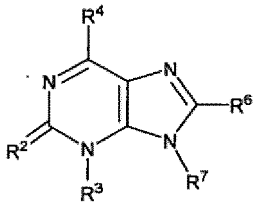 (9)

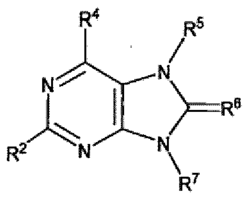 (10) 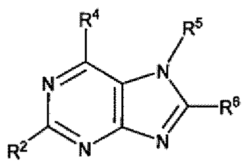 (11) 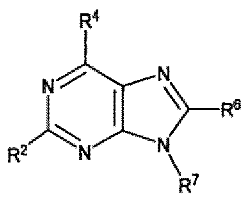 (12)

where
$R^2$, $R^4$ and $R^6$ which form double bonds with carbon atoms in the condensation polycyclic heterocyclic ring represent oxygen atoms or sulfur atoms and $R^2$, $R^4$ and $R^6$ which form single bonds with carbon atoms in the condensation polycyclic heterocyclic ring and $R^1$, $R^3$, $R^5$ and $R^7$ which bond with nitrogen atoms in the condensation polycyclic heterocyclic ring represent alkyl groups of 1 to 4 carbon atoms, haloalkyl groups of 1 to 4 carbon atoms, acylalkyl groups of 1 to 4 carbon atoms, allyl, propargyl, phenyl, benzyl, pyridyl, amino, pyrrolidylmethyl, trimethylsilyl, acetyl, trifluoroacetyl, chloromethyl, methoxymethyl, isocyanomethyl, methylsulfonyl, 2-(trimethylsilyl)-ethoxycarbonyloxy, bis(N,N'-alkyl)aminomethyl or bis(N,N'-alkyl)aminoethyl groups, alkoxy groups of 1 to 4 carbon atoms, fluorine-substituted alkoxy groups of 1 to 4 carbon atoms, nitrile groups, nitro groups, halogen atoms, saccharide residues or heterocyclic ring residues: with the proviso that $R^2$. $R^4$ and $R^6$ that form single bonds with carbon atoms in the condensation polycyclic heterocyclic ring are optionally hydrogen atoms, and their isomers.

[3] The nonaqueous electrolyte solution according to **[2]** above, wherein the condensation polycyclic heterocyclic ring compound is at least one selected from the group consisting of compounds represented by formulas (2), (5), (8) and (12), and their isomers.

[4] The nonaqueous electrolyte solution according to **[3]** above, wherein the condensation polycyclic heterocyclic ring compound is a compound represented by formula (2), or an isomer thereof.

[5] The nonaqueous electrolyte solution according to any one of [1] to **[4]** above, wherein the condensation polycyclic heterocyclic ring compound is caffeine.

[6] The nonaqueous electrolyte solution according to any one of [1] to **[5]** above, wherein the content of the condensation polycyclic heterocyclic ring compound is 0.01 weight% to 10 weight% based on the total weight of the nonaqueous electrolyte solution.

[7] The nonaqueous electrolyte solution according to any one of [1] to **[6]** above, wherein the lithium salt includes $LiPF_6$ and a lithium-containing imide salt.

[8] The nonaqueous electrolyte solution according to **[7]** above, wherein the content of the $LiPF_6$ is 0.01 mol/L or

greater and less than 0.1 mol/L with respect to the nonaqueous solvent.

[9] The nonaqueous electrolyte solution according to **[7]** or **[8]** above, wherein the molar ratio of the lithium-containing imide salt with respect to the $LiPF_6$ is greater than 10.

[10] The nonaqueous electrolyte solution according to any one of **[7]** to **[9]** above, wherein the lithium-containing imide salt includes lithium bis(fluorosulfonyl)imide.

**[11]** A nonaqueous secondary battery including the nonaqueous electrolyte solution according to any one of [1] to **[10]** above.

[12] The nonaqueous secondary battery according to **[11]** above, comprising:

a positive electrode that contains a lithium-phosphorus metal compound with an Fe-containing olivine crystal structure, and
a negative electrode that contains graphite or one or more elements selected from the group consisting of Ti, V, Sn, Cr, Mn, Fe, Co, Ni, Zn, Al, Si and B.

**[13]** The nonaqueous secondary battery according to **[12]** above, wherein the basis weight of the positive electrode per side is 15 $mg/cm^2$ or greater.

[ADVANTAGEOUS EFFECTS OF INVENTION]

According to the invention it is possible to provide a nonaqueous electrolyte solution with reduced degradation reaction in the nonaqueous secondary battery and reduced self-discharge at high temperatures above 60°C, and improved output characteristics and cycle performance in a wide range of temperatures, as well as a nonaqueous secondary battery comprising the solution. The invention can also provide, secondly, a nonaqueous electrolyte solution wherein the mixing ratio of lithium (Li) salts is controlled to avoid lack of negative electrode SEI formation and corrosion of the aluminum (Al) current collector while also inhibiting degradation reaction in the nonaqueous secondary battery caused by heat degradation of Li salts, and which is able to improve the output characteristic of the nonaqueous secondary battery in a wide range of temperatures, and to improve the long-term durability and cycle performance at high temperatures above 60°C, as well as a nonaqueous secondary battery comprising the solution.

BRIEF DESCRIPTION OF DRAWINGS

Fig. 1 is a plan view schematically showing an example of a nonaqueous secondary battery according to an embodiment of the invention.
Fig. 2 is a cross-sectional view of the nonaqueous secondary battery of Fig. 1 along line A-A.

DESCRIPTION OF EMBODIMENTS

An embodiment for carrying out the invention (hereunder referred to simply as "this embodiment") will now be explained in detail. Throughout the present specification, the numerical ranges indicated as "... to ..." include the bounding numerical values.

<First embodiment>

The nonaqueous electrolyte solution of the first embodiment, and a nonaqueous secondary battery that includes it, will now be described. By using a nonaqueous secondary battery according to this embodiment it is possible to provide a nonaqueous electrolyte solution with reduced degradation reaction in the nonaqueous secondary battery and reduced self-discharge at high temperatures above 60°C, as a result of reduced decomposition reaction of the nonaqueous electrolyte solution components by active oxygen species generated during the electrochemical reaction, resulting in improved output characteristics and cycle performance in a wide range of temperatures, as well as a nonaqueous secondary battery comprising the solution.

<1. Nonaqueous electrolyte solution>

A "nonaqueous electrolyte solution" for the first embodiment means an electrolyte solution comprising water at 1 weight% or lower with respect to the entire amount of the nonaqueous electrolyte solution. The nonaqueous electrolyte solution of this embodiment preferably contains as little water as possible, but it may contain a very trace amount of water in a range that does not interfere with solving the problem of the invention. The water content is 300 ppm by weight or lower and preferably 200 ppm by weight or lower, with respect to the entire amount of the nonaqueous electrolyte solution. The other constituent elements of the nonaqueous electrolyte solution may be appropriately selected from among constituent materials for known nonaqueous electrolyte solutions used in lithium ion batteries, so long as the construction still solves the problem of the invention.

The nonaqueous electrolyte solution of this embodiment can be produced by using any desired means to mix a lithium salt and various additives described below (also referred to herein simply as "additives") with a nonaqueous solvent. Here, the term "additives" generally includes electrode-protecting additives, condensation polycyclic heterocyclic ring compounds and other optional additives, and their contents are specified below.

Unless otherwise specified, the contents of the compounds in the nonaqueous solvent are mixing ratios in vol% with respect to the total amount of each of the components of the nonaqueous solvent, for the components listed under <2-1. Nonaqueous solvent> and the electrode-protecting additives listed under <2-3. Electrode-protecting additive>, or mixing ratios as number of moles per 1 L of nonaqueous solvent, for the lithium salts listed under <2-2. Lithium salt>, or mixing ratios in parts by weight where the total amount of the lithium salts and nonaqueous solvent is 100 parts by weight, for <2-4. Other optional additives> and <2-5. Condensation polycyclic heterocyclic ring compounds

For this embodiment, when the electrolyte solution includes a compound other than the compounds specifically mentioned under 2-1 to 2-4 above, and the compound is a liquid at ordinary temperature (25°C), it is treated in the same manner as the nonaqueous solvent, and the content represents the mixing ratio in vol% with respect to the total amount of the components composing the nonaqueous solvent (including the compound). When the compound is a solid at ordinary temperature (25°C), the content represents the mixing ratio as parts by weight with respect to 100 parts by weight as the total amount of the lithium salt and nonaqueous solvent.

<2-1. Nonaqueous solvent>

The term "nonaqueous solvent" for this embodiment refers to the element of the nonaqueous electrolyte solution after the lithium salt and additives have been removed. When an electrode-protecting additive is included in the nonaqueous electrolyte solution, the "nonaqueous solvent" is the element of the nonaqueous electrolyte solution after the lithium salt and additives other than the electrode-protecting additive have been removed.

The nonaqueous solvent of the nonaqueous electrolyte solution of the embodiment contains acetonitrile. Acetonitrile improves the ionic conductivity of the nonaqueous electrolyte solution and can therefore increase the diffusibility of lithium ions in the battery. Therefore, even with a positive electrode having a thicker positive electrode active material layer for an increased loading weight of the positive electrode active material, during discharge under high load it will be possible for lithium ions to satisfactorily diffuse even into the region near the current collector that is difficult for lithium ions to reach. This will allow sufficient capacity to be obtained even under high load discharge, so that a nonaqueous secondary battery with an excellent load characteristic can be obtained.

Moreover, by having the nonaqueous solvent contain acetonitrile, it is possible to increase the quick charging property of the nonaqueous secondary battery. During constant current (CC)-constant voltage (CV) charge of a nonaqueous secondary battery, the capacity per unit time during the CC charge period is greater than the charge capacity per unit time during the CV charge period. When acetonitrile is used as the nonaqueous solvent, it is possible to widen the region of CC charge (lengthen the time for CC charge), while also increasing the charging current, thus making it possible to significantly shorten the time from initial charge to full charge state of the nonaqueous secondary battery.

Because acetonitrile is easily susceptible to electrochemical reductive decomposition, it is preferred to add another solvent (for example, an aprotic solvent other than acetonitrile) together with acetonitrile as the nonaqueous solvent, and/or an electrode-protecting additive for formation of negative electrode SEI.

The acetonitrile content is 5 to 95 vol% with respect to the total nonaqueous solvent, from the viewpoint of increasing the ionic conductivity. The acetonitrile content is preferably 20 vol% or greater or 30 vol% or greater, and more preferably 40 vol% or greater, with respect to the total amount of the nonaqueous solvent. The value is also preferably 85 vol% or lower and more preferably 66 vol% or lower. If the acetonitrile content is 5 vol% or greater with respect to the total amount of the nonaqueous solvent, the ionic conductivity will increase tending to produce a high output characteristic, and dissolution of the lithium salt can also be accelerated. Since the additives mentioned below inhibit increase in the internal resistance of the battery, an acetonitrile content in this range for the nonaqueous solvent tends to maintain the excellent performance of the acetonitrile while also further improving the high temperature cycle characteristic and other battery characteristics. This tendency is even more pronounced when the positive electrode

used is a lithium-phosphorus metal oxide with an Fe-containing olivine crystal structure, or a positive electrode active material having a Ni ratio of 0.1 to 0.5 in the lithium-containing metal oxide. An acetonitrile content of 95 vol% or lower with respect to the total amount of nonaqueous solvent is preferred from the viewpoint of electrode protection. If the acetonitrile content in the nonaqueous solvent is within this range, it will be possible to add a sufficient amount of electrode-protecting additive for formation of negative electrode SEI, while maintaining the excellent performance of acetonitrile and keeping stable operation.

When the positive electrode active material used has a Ni molar ratio of Ni in the transition metal in the lithium-containing metal oxide (hereunder referred to as "Ni ratio") of greater than 0.5 and 0.7 or lower, the acetonitrile content is more preferably 5 vol% or greater and even more preferably 20 vol% or greater or 25 vol%, with respect to the total amount of the nonaqueous solvent. The value is also more preferably 60 vol% or lower, even more preferably 55 vol% or lower and yet more preferably 50 vol% or lower.

When the positive electrode active material used has a Ni ratio of higher than 0.7, the acetonitrile content is more preferably 5 vol% or greater and even more preferably 10 vol% or greater or 15 vol%, with respect to the total amount of the nonaqueous solvent. The value is also more preferably 50 vol% or lower, even more preferably 40 vol% or lower and yet more preferably 30 vol% or lower.

A positive electrode with a Ni ratio of 0.6 or higher produces various forms of degradation as the positive electrode active material layer cracks at the interfaces between the primary particles of the positive electrode active material, due to expansion and contraction of the positive electrode active material as charge-discharge takes place. When degradation proceeds, the positive electrode active material undergoes spinel transition and is no longer able to maintain its crystal structure, resulting in impaired battery performance. If the acetonitrile content in the nonaqueous solvent is within the range specified above, it is possible to maintain the excellent performance of the acetonitrile while inhibiting degradation of the positive electrode active material during charge-discharge in high-temperature environments, even when the nonaqueous secondary battery uses a positive electrode active material having a high nickel ratio.

It is also possible to use alcohols such as methanol or ethanol, or aprotic solvents, for example, in addition to acetonitrile. Aprotic solvents are preferred as nonaqueous solvents among these. The nonaqueous solvent may also contain a solvent other than an aprotic solvent, within a range that does not interfere with solving the problem of the invention.

Specific examples of aprotic solvents other than acetonitrile that may be used include cyclic carbonates. Examples of cyclic carbonates include carbonates such as ethylene carbonate, propylene carbonate, 1,2-butylene carbonate, *trans*-2,3-butylene carbonate, *cis*-2,3-butylene carbonate, 1,2-pentylene carbonate, *trans*-2,3-pentylene carbonate, *cis*-2,3-pentylene carbonate, vinylene carbonate, 4,5-dimethylvinylene carbonate and vinylethylene carbonate; fluorinated cyclic carbonates such as 4-fluoro-1,3-dioxolan-2-one, 4,4-difluoro-1,3-dioxolan-2-one, *cis*-4,5-difluoro-1,3-dioxolan-2-one, *trans*-4,5-difluoro-1,3-dioxolan-2-one, 4,4,5-trifluoro-1,3-dioxolan-2-one, 4,4,5,5-tetrafluoro-1,3-dioxolan-2-one and 4,4,5-trifluoro-5-methyl-1,3-dioxolan-2-one; and lactones such as by γ-butyrolactone, γ-valerolactone, γ-caprolactone, δ-valerolactone, δ-caprolactone and ε-caprolactone.

Specific examples of aprotic solvents include sulfur compounds such as ethylene sulfite, propylene sulfite, butylene sulfite, pentene sulfite, sulfolane, 3-sulfolene, 3-methylsulfolane, 1,3-propanesultone, 1,4-butanesultone, 1-propene-1,3-sultone, dimethyl sulfoxide, tetramethylene sulfoxide and ethyleneglycol sulfite; and cyclic ethers such as tetrahydrofuran, 2-methyltetrahydrofuran, 1,4-dioxane and 1,3-dioxane.

Specific examples of aprotic solvents include chain carbonates. Examples of chain carbonates include chain carbonates such as ethylmethyl carbonate, dimethyl carbonate, diethyl carbonate, methylpropyl carbonate, methylisopropyl carbonate, dipropyl carbonate, methylbutyl carbonate, dibutyl carbonate and ethylpropyl carbonate; and chain fluorinated carbonates such as trifluorodimethyl carbonate, trifluorodiethyl carbonate and trifluoroethylmethyl carbonate.

Specific examples of aprotic solvents include mononitriles such as propionitrile, butyronitrile, valeronitrile, benzonitrile and acrylonitrile; alkoxy group-substituted nitriles such as methoxyacetonitrile and 3-methoxypropionitrile; dinitriles such as malononitrile, succinonitrile, glutarornitrile, adiponitrile, 1,4-dicyanoheptane, 1,5-dicyanopentane, 1,6-dicyanohexane, 1,7-dicyanoheptane, 2,6-dicyanoheptane, 1,8-dicyanooctane, 2,7-dicyanooctane, 1,9-dicyanononane, 2,8-dicyanononane, 1,10-dicyanodecane, 1,6-dicyanodecane and 2,4-dimethylglutaronitrile; cyclic nitriles such as benzonitrile; chain esters such as methyl propionate; chain ethers such as dimethoxyethane, diethyl ether, 1,3-dioxolane, diglyme, triglyme and tetraglyme; fluorinated ethers such as RfA-ORB (where RfA represents a fluorine atom-containing alkyl group, and RB represents an organic group which may contain a fluorine atom); and ketones such as acetone, methyl ethyl ketone and methyl isobutyl ketone, as well as halogenated, such as fluorinated, forms of the foregoing.

They may also be used alone or as combinations of two or more types. Of these nonaqueous solvents, it is more preferred to use one or more cyclic carbonates or chain carbonates, from the viewpoint of improving the stability. A single type from among the cyclic carbonates and chain carbonates mentioned above may be selected for use, or two or more may be used in combination (for example, two or more of the cyclic carbonates, two or more of the chain carbonates, or two or more that include one or more of the cyclic carbonates and one or more of the chain carbonates). More preferred as cyclic carbonates are ethylene carbonate, propylene carbonate, vinylene carbonate or fluoroethylene

carbonate, and more preferred as chain carbonates are ethylmethyl carbonate, dimethyl carbonate or diethyl carbonate. It is more preferred to use a cyclic carbonate in order to increase the degree of ionization of the lithium salt that contributes to charge-discharge of the nonaqueous secondary battery. When a cyclic carbonate is used, the cyclic carbonate most preferably includes either or both vinylene carbonate and fluoroethylene carbonate.

<2-2. Lithium salt>

The nonaqueous electrolyte solution of this embodiment includes a lithium salt.
The lithium salt of the embodiment preferably includes a lithium-containing imide salt represented by the formula $LiN(SO_2C_mF_{2m+1})_2$ {where m is an integer of 0 to 8}.
The lithium salt of the embodiment may further include, with the lithium-containing imide salt, one or more selected from among fluorine-containing inorganic lithium salts, organic lithium salts and other lithium salts.

(Lithium-containing imide salt)

Specific examples of lithium-containing imide salts include either or both $LiN(SO_2F)_2$ and $LiN(SO_2CF_3)_2$.
Since the saturated concentration of a lithium-containing imide salt with respect to acetonitrile is higher than the saturated concentration of $LiPF_6$, it is preferred to include the lithium-containing imide salt at a molar concentration such that $LiPF_6$ <_ lithium-containing imide salt, from the viewpoint of inhibiting association and precipitation of the lithium salt and acetonitrile at low temperatures. A lithium-containing imide salt concentration of 0.5 mol to 3.0 mol per 1 L of the nonaqueous solvent is also preferred from the viewpoint of ensuring the ion supply rate to the nonaqueous electrolyte solution of the embodiment. With an acetonitrile-containing nonaqueous electrolyte solution including either or both $LiN(SO_2F)_2$ and $LiN(SO_2CF_3)_2$, it is possible to effectively inhibit reduction in the ion conductivity in the low-temperature region, and to obtain excellent low-temperature characteristics. By limiting the content in this manner it is therefore possible to more effectively inhibit resistance increase during high-temperature heating.

(Fluorine-containing inorganic lithium salt)

The lithium salt of this embodiment may include a fluorine-containing inorganic lithium salt. The term "fluorine-containing inorganic lithium salt" means an acetonitrile-soluble lithium salt that includes no carbon atoms in the anion and includes fluorine in the anion. The fluorine-containing inorganic lithium salt forms a passivation film on the surface of the positive electrode collector, which is excellent for inhibiting corrosion of the positive electrode collector.
Examples of fluorine-containing inorganic lithium salts include $LiPF_6$, $LiBF_4$, $LiAsF_6$, $Li_2SiF_6$, $LiSbF_6$ and $Li_2B_{12}F_bH_{12-b}$ {where b is an integer of 0 to 3}, and any one or more from among these may be selected for use.
Preferred fluorine-containing inorganic lithium salts are compounds that are compound salts of LiF and Lewis acids, among which fluorine-containing inorganic lithium salts with phosphorus atoms are more preferred because they readily release free fluorine. A typical fluorine-containing inorganic lithium salt is $LiPF_6$, which dissolves to release $PF_6$ anion. Fluorine-containing inorganic lithium salts with boron atoms are preferred because they can more easily trap excess free acid component that can potentially lead to battery degradation, and from this viewpoint, $LiBF_4$ is preferred.
The content of the fluorine-containing inorganic lithium salt in the nonaqueous electrolyte solution of this embodiment is preferably 0.01 mol or greater, more preferably 0.1 mol or greater and even more preferably 0.25 mol or greater, to 1 L of the nonaqueous solvent. If the fluorine-containing inorganic lithium salt content is within this range, the ionic conductivity will tend to increase, allowing a high output characteristic to be exhibited. The amount per 1 L of nonaqueous solvent is also preferably 2.8 mol or less, more preferably 1.5 mol or less and even more preferably 1.0 mol or less. If the fluorine-containing inorganic lithium salt content is within this range, the ionic conductivity will tend to increase, allowing a high output characteristic to be exhibited, while also helping to inhibit decrease in ionic conductivity due to increased viscosity at low temperature, and will tend to result in even more satisfactory high temperature cycle characteristics and other battery characteristics while maintaining the excellent performance of the nonaqueous electrolyte solution.
The content of the fluorine-containing inorganic lithium salt in the nonaqueous electrolyte solution of the embodiment may be 0.05 mol to 1.0 mol, for example, per 1 L of nonaqueous solvent.

(Organic lithium salt)

The lithium salt of this embodiment may also include an organic lithium salt. The term "organic lithium salt" used herein refers to a lithium salt that includes a carbon atom in the anion, is soluble in acetonitrile, and is not a lithium-containing imide salt.
Organic lithium salts include organic lithium salts having an oxalic acid structure. Specific examples of organic

lithium salts with an oxalic acid structure include organic lithium salts represented by $LiB(C_2O_4)_2$, $LiBF_2(C_2O_4)$, $LiPF_4(C_2O_4)$ and $LiPF_2(C_2O_4)_2$, among which one or more lithium salts represented by $LiB(C_2O_4)_2$ and $LiBF_2(C_2O_4)$ are preferred. More preferably, one or more of these is used together with a fluorine-containing inorganic lithium salt. An organic lithium salt with an oxalic acid structure may also be added to the negative electrode (negative electrode active material layer).

From the viewpoint of more satisfactorily ensuring that an effect is exhibited, the amount of organic lithium salt added to the nonaqueous electrolyte solution for this embodiment is preferably 0.005 mol or greater, more preferably 0.01 mol or greater, even more preferably 0.02 mol or greater and most preferably 0.05 mol or greater, per 1 L of nonaqueous solvent. However, excess addition of an organic lithium salt with an oxalic acid structure can potentially lead to precipitation from the nonaqueous electrolyte solution. Therefore, the amount of organic lithium salt with an oxalic acid structure added to the nonaqueous electrolyte solution is preferably less than 1.0 mol, more preferably less than 0.5 mol and even more preferably less than 0.2 mol, to 1 L of the nonaqueous solvent.

An organic lithium salt with an oxalic acid structure is poorly soluble in low-polarity organic solvents, and especially chain carbonates. The content of the organic lithium salt in the nonaqueous electrolyte solution of the embodiment may be 0.01 mol to 0.5 mol, for example, per 1 L of nonaqueous solvent.

An organic lithium salt with an oxalic acid structure sometimes contains trace amounts of lithium oxalate, and when mixed in with a nonaqueous electrolyte solution, it reacts with trace amounts of water in the other starting materials to result in precipitation of lithium oxalate. The lithium oxalate content in the nonaqueous electrolyte solution of this embodiment is preferably limited to a range of 500 ppm or lower.

(Other lithium salts)

The lithium salt of this embodiment may also include other additional lithium salts. Specific examples of other lithium salts include:

inorganic lithium salts without fluorine in the anion, such as $LiClO_4$, $LiAlO_4$, $LiAlCl_4$, $LiB_{10}Cl_{10}$ and lithium chloroborane;
organic lithium salts such as $LiCF_3SO_3$, $LiCF_3CO_2$, $Li_2C_2F_4(SO_3)_2$, $LiC(CF_3SO_2)_3$, $LiC_nF_{(2n+1)}SO_3$ ($n \geq 2$), lower aliphatic carboxylic acid Li salts, Li tetraphenylborate and $LiB(C_3O_4H_2)_2$;
organic lithium salts represented by $LiPF_n(C_pF_{2p+1})_{6-n}$ (e.g. $LiPF_5(CF_3)$) [where n is an integer of 1 to 5 and p is an integer of 1 to 8];
organic lithium salts represented by $LiBF_q(C_sF_{2s+1})_{4-q}$ (e.g. $LiBF_3(CF_3)$) [where q is an integer of 1 to 3 and s is an integer of 1 to 8];
multivalent anion-bonded lithium salts; and
organic lithium salts represented by:

the following formula (XXa):

$$LiC(SO_2R^{jj})(SO_2R^{kk})(SO_2R^{ll}) \qquad (XXa)$$

{where $R^{jj}$, $R^{kk}$ and $R^{ll}$ may be the same or different and each represents a perfluoroalkyl group of 1 to 8 carbon atoms},
the following formula (XXb):

$$LiN(SO_2OR^{mm})(SO_2OR^{nn}) \qquad (XXb)$$

{where $R^{mm}$ and $R^{nn}$ may be the same or different and each represents a perfluoroalkyl group of 1 to 8 carbon atoms}, and
the following formula (XXc):

$$LiN(SO_2R^{oo})(SO_2OR^{pp}) \qquad (XXc)$$

{where $R^{oo}$ and $R^{pp}$ may be the same or different and each represents a perfluoroalkyl group of 1 to 8 carbon atoms},

and any one or more of these may be used together with a fluorine-containing inorganic lithium salt.

The amount of other lithium salts added to the nonaqueous electrolyte solution may be appropriately set within a range of 0.01 mol to 0.5 mol, for example, per 1 L of the nonaqueous solvent.

<2-3. Electrode-protecting additive>

The nonaqueous electrolyte solution of this embodiment may also include an additive to protect the electrode ("electrode-protecting additive"). The electrode-protecting additive may essentially overlap with substances that serve as solvents for dissolution of the lithium salt (i.e. the nonaqueous solvent). The electrode-protecting additive is preferably a substance that contributes to improved performance of the nonaqueous electrolyte solution and nonaqueous secondary battery, but it may also be a substance that does not directly take part in the electrochemical reaction.

Specific examples of electrode-protecting additives include:

fluoroethylene carbonates such as 4-fluoro-1,3-dioxolan-2-one, 4,4-difluoro-1,3-dioxolan-2-one, *cis*-4,5-difluoro-1,3-dioxolan-2-one, *trans*-4,5-difluoro-1,3-dioxolan-2-one, 4,4,5-trifluoro-1,3-dioxolan-2-one, 4,4,5,5-tetrafluoro-1,3-dioxolan-2-one and 4,4,5-trifluoro-5-methyl-1,3-dioxolan-2-one;
unsaturated bond-containing cyclic carbonates such as vinylene carbonate, 4,5-dimethylvinylene carbonate and vinylethylene carbonate;
lactones such as γ-butyrolactone, γ-valerolactone, γ-caprolactone, δ-valerolactone, δ-caprolactone and ε-caprolactone;
cyclic ethers such as 1,4-dioxane; and
cyclic sulfur compounds such as ethylene sulfite, propylene sulfite, butylene sulfite, pentene sulfite, sulfolane, 3-sulfolene, 3-methylsulfolane, 1,3-propanesultone, 1,4-butanesultone and 1-propene-1,3-sultone and tetramethylene sulfoxide;

any of which may be used alone, or in combinations of two or more.

The content of the electrode-protecting additive in the nonaqueous electrolyte solution is preferably 0.1 to 30 vol%, more preferably 0.3 to 15 vol%, even more preferably 0.4 to 8 vol% and most preferably 0.5 to 6.5 vol%, with respect to the total amount of the nonaqueous solvent. For this embodiment, a larger electrode-protecting additive content more greatly inhibits degradation of the nonaqueous electrolyte solution. However, a lower electrode-protecting additive content improves the high output characteristic of the nonaqueous secondary battery in low temperature environments. By adjusting the electrode-protecting additive content within the range specified above, therefore, it is possible to exhibit the excellent performance based on the high ionic conductivity of the electrolyte solution without impairing the basic function of the nonaqueous secondary battery. By adjusting the nonaqueous electrolyte solution to this type of composition, it is possible to obtain even more satisfactory cycle performance, high output performance in low temperature environments, and other battery characteristics of the nonaqueous secondary battery.

Acetonitrile readily undergoes electrochemical reductive decomposition. Therefore, the electrode-protecting additives for formation of negative electrode SEI preferably include one or more cyclic polar aprotic solvents, and more preferably include one or more unsaturated bond-containing cyclic carbonates.

An unsaturated bond-containing cyclic carbonate is preferably vinylene carbonate, the vinylene carbonate content in the nonaqueous electrolyte solution being preferably 0.1 vol% to 4 vol%, more preferably 0.2 vol% or greater and less than 3 vol%, and even more preferably 0.5 vol% to 2.5 vol%. This will allow the low temperature durability to be more effectively improved and can provide a secondary battery with excellent low temperature performance.

Vinylene carbonate used as an electrode-protecting additive inhibits reductive decomposition reaction of acetonitrile on the negative electrode surface. Excess formation of a coating film, however, leads to lower low temperature performance. By adjusting the amount of vinylene carbonate added to within this range it is possible to inhibit reduction in the interfacial (coating film) resistance, and to inhibit cycle degradation at low temperature.

<2-4. Other optional additives>

In order to improve the charge-discharge cycle characteristic of the nonaqueous secondary battery and to increase the high temperature storage property and safety (such as preventing overcharge) for this embodiment, optional additives may also be added as appropriate to the nonaqueous electrolyte solution, which are selected from among sulfonic acid esters, diphenyl disulfide, cyclohexylbenzene, biphenyl, fluorobenzene, *tert*-butylbenzene, phosphoric acid esters [such as ethyldiethyl phosphonoacetate (EDPA): $(C_2H_5O)_2(P{=}O){-}CH_2(C{=}O)OC_2H_5$, tris(trifluoroethyl) phosphate (TFEP): $(CF_3CH_2O)_3P{=}O$, triphenyl phosphate (TPP): $(C_6H_5O)_3P{=}O$: $(CH_2{=}CHCH_2O)_3P{=}O$ and triallyl phosphate], nitrogen-containing cyclic compounds without steric hindrance around the unshared electron pair [such as pyridine, 1-methyl-1H-benzotriazole and 1-methylpyrazole], and derivatives of these compounds. Phosphoric acid esters, in particular, have effects of inhibiting secondary reactions while the nonaqueous electrolyte solution or battery is being stored.

The nonaqueous secondary battery of this embodiment undergoes partial decomposition of the nonaqueous electrolyte solution during initial charge, forming SEI on the negative electrode surface. In order to reinforce the negative electrode SEI, an acid anhydride may be added as an additive to the nonaqueous electrolyte solution. When acetonitrile

is included as a nonaqueous solvent, the strength of the negative electrode SEI tends to be lower with increasing temperature, but the negative electrode SEI is more actively reinforced by addition of an acid anhydride. Using an acid anhydride in this manner can effectively inhibit increase in internal resistance with time due to thermal history.

Specific examples of acid anhydrides include chain acid anhydrides such as acetic anhydride, propionic anhydride and benzoic anhydride; cyclic acid anhydrides such as malonic anhydride, succinic anhydride, glutaric anhydride, maleic anhydride, phthalic anhydride, 1,2-cyclohexanedicarboxylic anhydride, 2,3-naphthalenedicarboxylic anhydride and naphthalene-1,4,5,8-tetracarboxylic dianhydride; and mixed acid anhydrides having a structure in which different types of acids, such as two different carboxylic acids or a carboxylic acid and sulfonic acid, are combined by dehydrating condensation. They may also be used alone or as combinations of two or more types.

Because it is preferred to reinforce the negative electrode SEI before reductive decomposition of the nonaqueous solvent, the nonaqueous secondary battery of this embodiment preferably includes at least one cyclic acid anhydride to function early as an acid anhydride during initial charge. Such a cyclic acid anhydride may be of a single type or a combination of different types, and a cyclic acid anhydride other than one of these cyclic acid anhydrides may also be included. The cyclic acid anhydride preferably also includes at least one from among succinic anhydride, maleic anhydride and phthalic anhydride.

A nonaqueous electrolyte solution that includes at least one from among succinic anhydride, maleic anhydride and phthalic anhydride can form a strong SEI on the negative electrode and will more effectively inhibit increase in resistance during high-temperature heating. Most preferably, it includes succinic anhydride. This can inhibit secondary reactions while also more effectively forming a strong SEI on the negative electrode.

When the nonaqueous electrolyte solution of this embodiment contains an acid anhydride, the content is preferably in the range of 0.01 part by weight to 10 parts by weight, more preferably 0.05 part by weight to 1 part by weight and even more preferably 0.1 part by weight to 0.5 part by weight, with respect to 100 parts by weight of the nonaqueous electrolyte solution. If the acid anhydride content in the nonaqueous electrolyte solution is within this range, it will be possible to more effectively reinforce the negative electrode SEI, and to more effectively improve the high-temperature durability of the nonaqueous secondary battery using the acetonitrile electrolyte solution.

An acid anhydride is preferably added to the nonaqueous electrolyte solution. Since it is sufficient if the acid anhydride is able to interact in the nonaqueous secondary battery, it is sufficient if at least one battery element selected from the group consisting of the positive electrode, negative electrode and separator contains the acid anhydride. The method of adding the acid anhydride to the battery element may be, for example, addition of the acid anhydride to the battery element during fabrication of the battery element, or impregnation of the battery element with the acid anhydride by post-treatment such as coating onto the battery element, or dipping or spray-drying.

The content of other optional additives for this embodiment is preferably in the range of 0.01 weight% to 10 weight%, more preferably 0.02 weight% to 5 weight% and even more preferably 0.05 to 3 weight%, with respect to the total amount of the nonaqueous electrolyte solution. By adjusting the content of the other optional additives to within this range, even more satisfactory battery characteristics will tend to be imparted without impairing the basic function of the nonaqueous secondary battery.

<2-5. Condensation polycyclic heterocyclic ring compound>

The nonaqueous electrolyte solution of this embodiment comprises a compound (condensation polycyclic heterocyclic ring compound) having a structure satisfying the following conditions 1 to 5:

1. being a condensation polycyclic heterocyclic ring compound,
2. containing a pyrimidine backbone in the condensation polycyclic heterocyclic ring,
3. containing 3 or more nitrogen atoms in the condensation polycyclic heterocyclic ring,
4. containing 5 or more sp2 carbons in the condensation polycyclic heterocyclic ring, and
5. having no hydrogen atoms bonded to the nitrogen atoms in the condensation polycyclic heterocyclic ring. It is preferred to use a purine derivative as the condensation polycyclic heterocyclic ring compound. A purine derivative is a compound in which the basic backbone is a bicyclic heterocyclic ring having an imidazole ring bonded to a pyrimidine backbone. More preferably, the condensation polycyclic heterocyclic ring compound contains at least one selected from the group consisting of compounds represented by the following formulas (1) to (12):

[Chemical Formula 2]

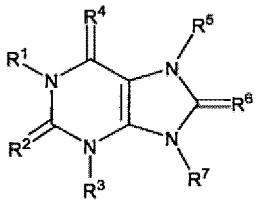 (1) 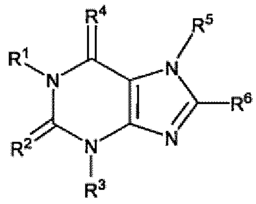 (2) 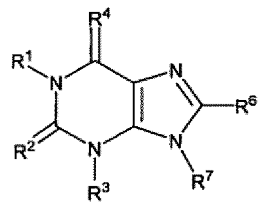 (3)

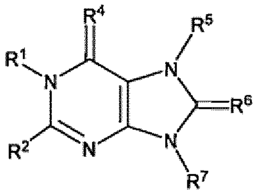 (4) 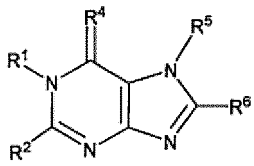 (5) 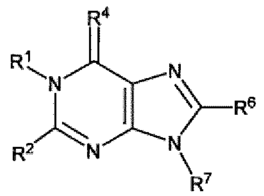 (6)

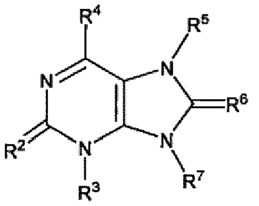 (7) 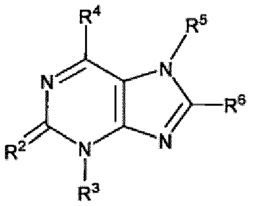 (8) 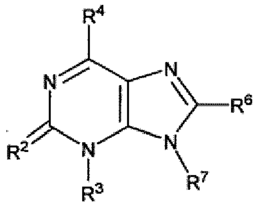 (9)

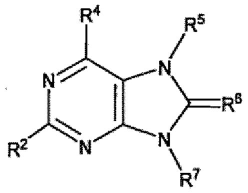 (10) 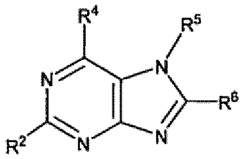 (11) 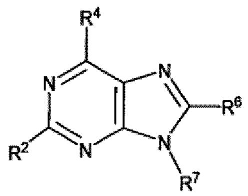 (12)

where $R^2$, $R^4$ and $R^6$ which form double bonds with carbon atoms in the condensation polycyclic heterocyclic ring represent oxygen atoms or sulfur atoms, and $R^2$, $R^4$ and $R^6$ which form single bonds with carbon atoms in the condensation polycyclic heterocyclic ring and $R^1$, $R^3$, $R^5$ and $R^7$ which bond with nitrogen atoms in the condensation polycyclic heterocyclic ring represent alkyl groups of 1 to 4 carbon atoms, haloalkyl groups of 1 to 4 carbon atoms, acylalkyl groups of 1 to 4 carbon atoms, allyl, propargyl, phenyl, benzyl, pyridyl, amino, pyrrolidylmethyl, trimethylsilyl, nitrile, acetyl, trifluoroacetyl, chloromethyl, methoxymethyl, isocyanomethyl, methylsulfonyl, 2-(trimethylsilyl)-ethoxycarbonyloxy, bis(N,N'-alkyl)aminomethyl or bis(N,N'-alkyl)aminoethyl groups, alkoxy groups of 1 to 4 carbon atoms, fluorine-substituted alkoxy groups of 1 to 4 carbon atoms, nitrile groups, nitro groups, halogen atoms, saccharide residues or heterocyclic ring residues: with the proviso that $R^2$, $R^4$ and $R^6$ that form single bonds with carbon atoms in the condensation polycyclic heterocyclic ring are optionally hydrogen atoms, and their isomers.

The following are specific examples of condensation polycyclic heterocyclic ring compounds for this embodiment. They may also be used alone or as combinations of two or more types.

[Chemical Formula 3]

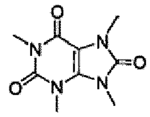 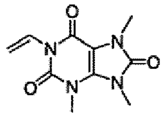 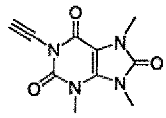 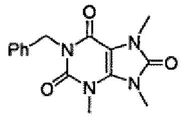 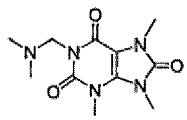

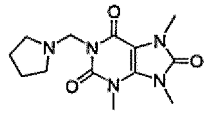 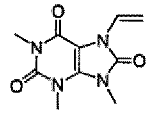 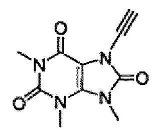 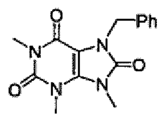 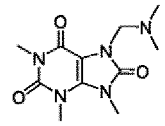

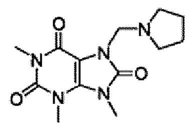 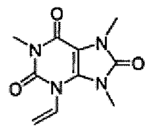 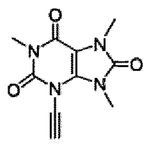 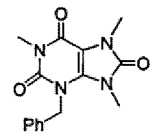 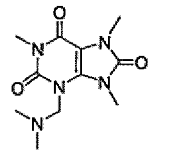

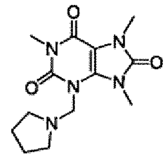 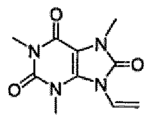 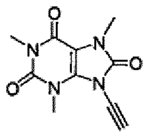 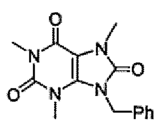 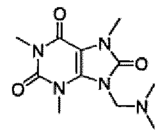

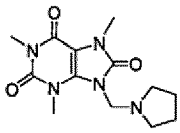 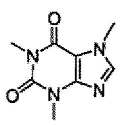 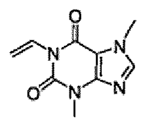 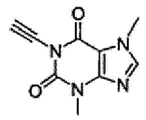 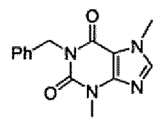

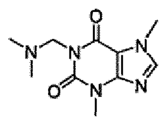 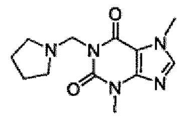 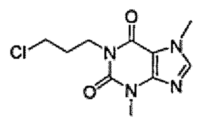 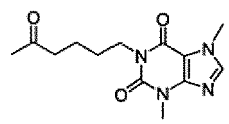 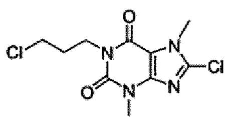

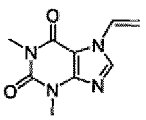 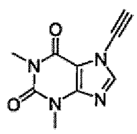 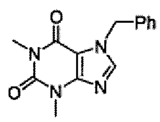 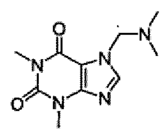 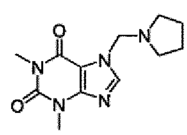

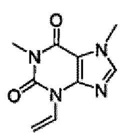 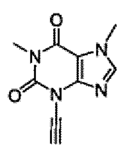 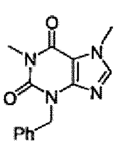 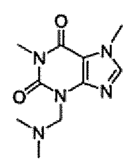 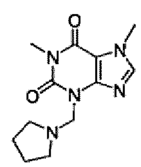

[Chemical Formula 4]

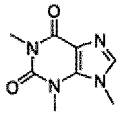 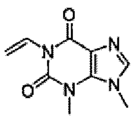 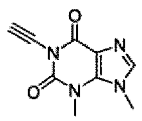 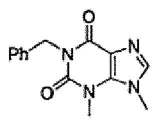 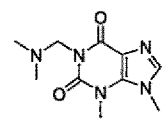

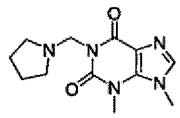 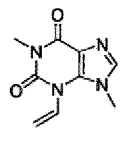 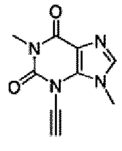 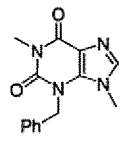 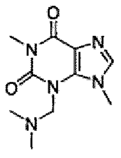

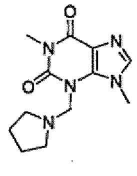 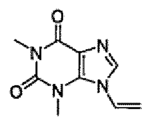 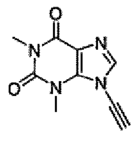 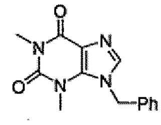 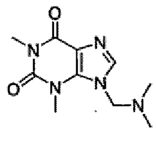

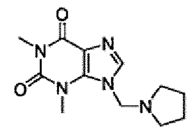 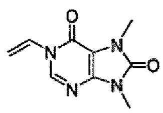 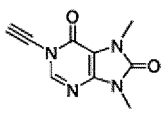 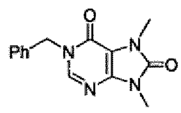 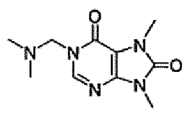

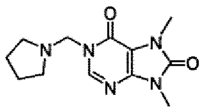 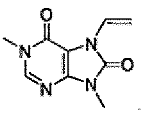 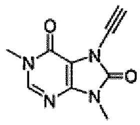 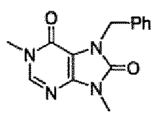 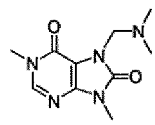

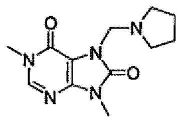 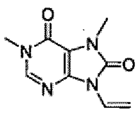 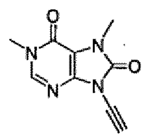 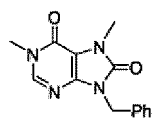 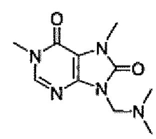

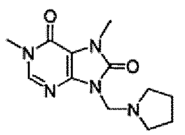 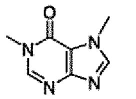 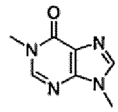 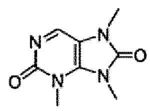 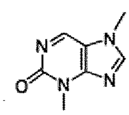

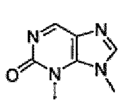 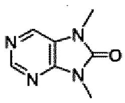 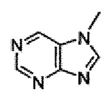 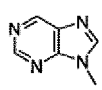

Of the examples of these formulas, the condensation polycyclic heterocyclic ring compound is preferably at least one selected from the group consisting of compounds represented by formulas (2), (5), (8) and (12), and their isomers, more preferably compounds represented by formula (2) and, among the compounds represented by formula (2), even more preferably caffeine.

The content of the condensation polycyclic heterocyclic ring compound in the electrolyte solution of this embodiment is preferably 0.01 weight% or greater, more preferably 0.05 weight% or greater and even more preferably 0.1 weight% or greater, based on the total weight of the electrolyte solution. For this embodiment, the condensation polycyclic heterocyclic ring compound inhibits formation of complex cations formed from the transition metal and acetonitrile. The nonaqueous secondary battery containing the condensation polycyclic heterocyclic ring compound therefore exhibits an excellent load characteristic, and inhibited increase in internal resistance after repeated charge-discharge cycling. The content of the condensation polycyclic heterocyclic ring compound in the electrolyte solution of this embodiment is also preferably 10 weight% or lower, more preferably 5 weight% or lower, even more preferably 1 weight% or lower and

yet more preferably 0.5 weight% or lower, based on the total weight of the electrolyte solution. By adjusting the content of the condensation polycyclic heterocyclic ring compound to within this range for the embodiment, it is possible to inhibit complex cation-generating reactions on the electrode surface and lower increase in internal resistance with charge-discharge, without impairing the basic function of the nonaqueous secondary battery. Moreover, by adjusting the electrolyte solution of this embodiment to within the range specified above it is possible to obtain even more satisfactory cycle performance, high output performance in low temperature environments and the other battery characteristics of the nonaqueous secondary battery that is obtained.

While the action mechanism by which the condensation polycyclic heterocyclic ring compound in the electrolyte solution of this embodiment inhibits degradation of the electrolyte solution is not completely understood, one mechanism has been reported, in which the sp2 carbons at the 3 locations on the 5-membered ring in the imidazolyl ring adjacent to the pyrimidine backbone of the polar solvent act as reaction sites to exhibit an antioxidant effect. Since active oxygen species are generated during electrochemical reaction on the positive electrode surface, it is similar to an oxidative environment in the polar solvent, and a similar effect may be expected even with the additives of the electrolyte solution for a nonaqueous secondary battery. The phenomenon is particularly notable at the positive electrode that contains Ni which is easily susceptible to phase transition, and is even more notable at a positive electrode that uses a positive electrode active material with a high Ni ratio. It is especially notable at a positive electrode using a positive electrode active material with a Ni ratio of 0.6 or higher, and extremely notable at a positive electrode using a positive electrode active material with a Ni ratio of 0.7 or higher. In addition, when a positive electrode having such a high Ni ratio is combined with a nonaqueous electrolyte solution having high ionic conductivity, excess Li is removed from the positive electrode during charge, making release of active oxygen with structural change even more notable. Variation in diffusion inside the positive electrode also locally amplifies this tendency, so that the effect of Li withdrawal is more influential on a positive electrode with a higher basis weight, which tends to have more variation in diffusion inside the positive electrode. It is therefore surmised that a condensation polycyclic heterocyclic ring compound represented by any of formulas (1) to (12) can inhibit degradation reaction of the electrolyte solution by active oxygen species, allowing an even higher effect to be obtained when a positive electrode active material with a high Ni ratio is used. One of the mechanisms by which an antioxidant effect is exhibited is accelerated reaction in the presence of iron (Fe) ions. From this viewpoint, therefore, a high effect can be expected when using a positive electrode containing iron (Fe) ion, and a particularly high effect can be expected to be obtained upon prolonged exposure to conditions in which iron (Fe) elutes from the positive electrode, such as high temperatures of 60°C or higher. The condensation polycyclic heterocyclic ring compound also has a pyrimidine backbone or imidazolyl ring, and thus has a Lewis base property due to the unshared electron pair of the nitrogen atom. It is therefore conjectured that interaction between the condensation polycyclic heterocyclic ring compound and the powerful Lewis acid such as $PF_5$, as a product of decomposition of the electrolyte salt, stabilizes the $PF_5$ and allows generation of hydrofluoric acid (HF) from $PF_5$ to be inhibited. It is further conjectured that interaction with powerful Bronsted acids such as HF has an effect of inhibiting metal elution from the battery elements.

<Overall construction of nonaqueous secondary battery>

The nonaqueous electrolyte solution of this embodiment may be used in a nonaqueous secondary battery. A nonaqueous secondary battery of the embodiment is not particularly restricted in terms of its negative electrode, positive electrode, separator and battery exterior.

The nonaqueous secondary battery of the embodiment may be a lithium ion battery comprising a positive electrode that contains a positive electrode material capable of storing and releasing lithium ions, as the positive electrode active material, and a negative electrode that contains a negative electrode material capable of storing and releasing lithium ions, as the negative electrode active material, and/or metal lithium.

More specifically, the nonaqueous secondary battery of the embodiment may be the nonaqueous secondary battery illustrated in Figs. 1 and 2. Fig. 1 is a plan view schematically showing a nonaqueous secondary battery, and Fig. 2 is a cross-sectional view along line A-A of Fig. 1.

The nonaqueous secondary battery 100 shown in Fig. 1 and Fig. 2 is composed of a pouch-type cell. The nonaqueous secondary battery 100 houses a stacked electrode structure comprising a positive electrode 150 and a negative electrode 160 stacked via a separator 170, and a nonaqueous electrolyte solution (not shown), in the space 120 of a battery exterior 110 composed of two aluminum laminate films. The battery exterior 110 is sealed by heat fusion of the upper and lower aluminum laminate films at the outer periphery. The nonaqueous electrolyte solution is impregnated in the layered product obtaining by stacking the positive electrode 150, separator 170 and negative electrode 160 in that order. In Fig. 2, however, in order to avoid complicating the drawing, the layers forming the battery exterior 110 and the layers of the positive electrode 150 and negative electrode 160 are not indicated separately.

The aluminum laminate films composing the battery exterior 110 are preferably coated with a polyolefin-based resin on both sides of the aluminum foil.

The positive electrode 150 is connected with a positive electrode lead 130 in the nonaqueous secondary battery

100. While not shown, the negative electrode 160 is also connected to a negative electrode lead 140 in the nonaqueous secondary battery 100. The positive electrode lead 130 and negative electrode lead 140 each have one side extending outside of the battery exterior 110 so as to be connectable to external devices, and their ionomer portions are heat-condensation together with one side of the battery exterior 110.

In the nonaqueous secondary battery 100 shown in Figs. 1 and 2, the positive electrode 150 and negative electrode 160 each have a single stacked electrode structure, but the number of stacked positive electrode 150 and negative electrode 160 layers may be increased as appropriate for the capacity design. When the stacked electrode structure has multiple layers of the positive electrode 150 and negative electrode 160, tabs for the same pole may be joined by welding or the like and then joined to a single lead also by welding, and extended outside of the battery. Tabs for the same pole may be composed of the exposed portions of the current collectors, or they may be composed of metal fragments welded to the exposed portions of the current collectors.

The positive electrode 150 is composed of a positive electrode active material layer fabricated from a positive electrode mixture, and a positive electrode collector. The negative electrode 160 is composed of a negative electrode active material layer fabricated from a negative electrode mixture, and a negative electrode collector. The positive electrode 150 and negative electrode 160 are disposed with the positive electrode active material layer and negative electrode active material layer facing each other across the separator 170.

Each of the elements may be materials used in conventional lithium ion batteries, so long as they satisfy the conditions for this embodiment. Further details regarding each of the elements of the nonaqueous secondary battery will now be explained.

<Positive electrode>

The positive electrode 150 is composed of a positive electrode active material layer fabricated from a positive electrode mixture, and a positive electrode collector. The positive electrode mixture comprises a positive electrode active material, and if necessary also a conductive aid and a binder.

The positive electrode active material layer comprises, as the positive electrode active material, a material capable of storing and releasing lithium ions. Such materials can produce high voltage and high energy density.

Examples for the positive electrode active material include positive electrode active materials comprising at least one transition metal element selected from the group consisting of Ni, Mn and Co, preferred among which are one or more Li-containing metal oxides selected from among lithium (Li)-containing metal oxides represented by the following formula ($a^t$):

$$Li_pNi_qCo_rMn_sM_tO_u \qquad (a^t)$$

{where M is at least one metal selected from the group consisting of Al, Sn, In, Fe, V, Cu, Mg, Ti, Zn, Mo, Zr, Sr and Ba, the ranges: $0 < p < 1.3$, $0 < q < 1.2$, $0 < r < 1.2$, $0 \leq s < 0.5$, $0 \leq t < 0.3$, $0.7 \leq q + r + s + t \leq 1.2$, $1.8 < u < 2.2$ are satisfied, and p is a value determined by the charge-discharge state of the battery}.

Specific examples of positive electrode active materials include lithium cobaltic acid compounds such as $LiCoO_2$; lithium manganic acid compounds such as $LiMnO_2$, $LiMn_2O_4$ and $Li_2Mn_2O_4$; lithium nickelic acid compounds such as $LiNiO_2$; and lithium-containing complex metal oxides represented by $Li_zMO_2$ compounds (where M represents two or more metal elements selected from the group consisting of Ni, Mn, Al and Mg, and z represents a value greater than 0.9 and less than 1.2), such as $LiNi_{1/3}Co_{1/3}Mn_{1/3}O_2$, $LiNi_{0.5}Co_{0.2}Mn_{0.3}O_2$ and $LiNi_{0.8}Co_{0.2}O_2$.

In particular, the Ni-containing ratio q for the Li-containing metal oxide represented by formula ($a^t$) is preferably such that $0.5 < q < 1.2$, in order to both reduce the amount of rare metal (Co) used and obtain high energy density. Examples of such positive electrode active materials include lithium-containing complex metal oxides such as $LiNi_{0.6}Co_{0.2}Mn_{0.2}O_2$, $LiNi_{0.75}LiNi_{0.8}Co_{0.1}Mn_{0.1}O_2$, $LiNi_{0.85}Co_{0.075}Mn_{0.075}O_2$, $LiNi_{0.8}Co_{0.15}Al_{0.05}O_2$, $LiNi_{0.81}Co_{0.1}Al_{0.09}O_2$ and $LiNi_{0.85}Co_{0.1}Al_{0.05}O_2$.

A higher Ni-content ratio of the Li-containing metal oxide, on the other hand, will tend to accelerate degradation at low voltage. A layered rock salt type positive electrode active material represented by formula ($a^t$) intrinsically has active sites that cause oxidative degradation of the electrolyte solution. The active sites often unintentionally consume the electrode-protecting additive.

The decomposition product of the additives that have been incorporated and deposited on the positive electrode side not only cause increased internal resistance for the nonaqueous secondary battery, but also accelerate degradation of the lithium salt. Particularly when $LiPF_6$ is present as a lithium salt, HF is generated by degradation, accelerating elution of the transition metal. In a nonaqueous electrolyte solution containing acetonitrile as the nonaqueous solvent, a complex of the metal cation and acetonitrile is formed, accelerating degradation of the battery.

Degradation of the electrode-protecting additive and lithium salt also makes it impossible to sufficiently protect the negative electrode surface, which is the original purpose. Particularly with a nonaqueous electrolyte solution containing acetonitrile as the nonaqueous solvent, reductive decomposition of the acetonitrile proceeds when the negative electrode surface is not sufficiently protected, leading to the fatal issue of drastic loss of battery performance.

In order to inactivate the active sites that essentially cause oxidative degradation of the nonaqueous electrolyte solution, it is important to include components that control Jahn-Teller distortion and function as neutralizers. It is therefore preferred to add at least one metal selected from the group consisting of Al, Sn, In, Fe, V, Cu, Mg, Ti, Zn, Mo, Zr, Sr and Ba to the positive electrode active material.

For the same reason, the surface of the positive electrode active material is preferably covered by a compound containing at least one metal element selected from the group consisting of Zr, Ti, Al and Nb. The surface of the positive electrode active material is more preferably covered by an oxide containing at least one metal element selected from the group consisting of Zr, Ti, Al and Nb. Most preferably, the surface of the positive electrode active material is covered by at least one oxide selected from the group consisting of $ZrO_2$, $TiO_2$, $Al_2O_3$, $NbO_3$ and $LiNbO_2$, since this will avoid inhibiting permeation of lithium ions.

In a nonaqueous secondary battery of this embodiment it is preferred to use a lithium-phosphorus metal oxide having an olivine crystal structure that includes an iron (Fe) atom, and it is more preferred to use a lithium-phosphorus metal oxide having an olivine structure represented by the following formula (Xba):

$$Li_WM^{II}PO_4 \qquad (Xba)$$

{where $M^{II}$ represents one or more transition metal elements, including at least one transition metal element that contains Fe, and the value of w is determined by the charge-discharge state of the battery and represents a value of 0.05 to 1.10}. For structural stabilization, these lithium-containing metal oxides may be ones having a portion of the transition metal element replaced with Al, Mg, or another transition metal element, or having the metal elements added at the grain boundaries, or having some of the oxygen atoms replaced with fluorine atoms, or having another positive electrode active material covering at least part of the positive electrode active material surface.

Examples of positive electrode active materials include phosphoric acid metal oxides containing lithium and a transition metal element, and silicic acid metal oxides containing lithium and a transition metal element. From the viewpoint of obtaining higher voltage, a lithium-containing metal oxide is preferably a phosphoric acid metal oxide containing lithium and at least one transition metal element selected from the group consisting of Co, Ni, Mn, Fe, Cu, Zn, Cr, V and Ti, and from the viewpoint of the lithium-phosphorus metal oxide represented by formula (Xba) above, it is more preferably a phosphoric acid metal oxide containing Li and Fe.

Lithium-phosphorus metal oxides to be used that are different from lithium-phosphorus metal oxides represented by formula (Xba) include compounds represented by the following formula (Xa):

$$Li_vM^ID_2 \qquad (Xa)$$

{where D represents a chalcogen element, $M^I$ represents one or more transition metal elements that include at least one type of transition metal element, and the value of v is determined by the charge-discharge state of the battery and represents a value of 0.05 to 1.10}.

The positive electrode active material of this embodiment may be one of the lithium-containing metal oxides mentioned above alone, or optionally another positive electrode active material may be used together with a lithium-containing metal oxide.

Examples of other positive electrode active materials include metal oxides or metal chalcogenides having tunnel structures and laminar structures; sulfur; and conductive polymers. Examples of metal oxides or metal chalcogenides having tunnel structures or laminar structures include oxides, sulfides and selenides of metals other than lithium, such as $MnO_2$, $FeO_2$, $FeS_2$, $V_2O_5$, $V_6O_{13}$, $TiO_2$, $TiS_2$, $MoS_2$ and $NbSe_2$. Examples of conductive polymers include conductive polymers such as polyaniline, polythiophene, polyacetylene and polypyrrole.

The other positive electrode active material used may be a single type or a combination of two or more types. The positive electrode active material layer preferably contains at least one transition metal element selected from among Ni, Mn and Co, since this will allow reversible and stable storage and release of lithium ions and can provide high energy density.

When a lithium-containing metal oxide and another positive electrode active material are used together as the positive electrode active material, the proportion in which both are used is preferably 80 weight% or greater and more preferably 85 weight% or greater, as the amount of lithium-containing metal oxide used with respect to the total positive electrode active material.

The positive electrode active material layer is formed by a procedure in which a positive electrode mixture-containing slurry in which a positive electrode mixture comprising a mixture of the positive electrode active material, and a conductive aid and binder if necessary, is dispersed in a solvent (solvent removal), which is coated and dried (solvent removal) onto the positive electrode collector, and pressing is carried out if necessary. The solvent used may be any

publicly known one. Examples include N-methyl-2-pyrrolidone, dimethylformamide, dimethylacetamide and water.

Examples of conductive aids include carbon blacks such as acetylene black and Ketchen black; carbon fibers; and graphite. The content ratio of the conductive aid is preferably 10 parts by weight or lower and more preferably 1 to 5 parts by weight with respect to 100 parts by weight of the positive electrode active material.

Examples of binders include polyvinylidene fluoride (PVDF), polytetrafluoroethylene (PTFE), polyacrylic acid, styrene-butadiene rubber and fluorine rubber. The content ratio of the binder is preferably 6 parts by weight or lower and more preferably 0.5 to 4 parts by weight with respect to 100 parts by weight of the positive electrode active material.

The positive electrode collector is composed of a metal foil such as aluminum foil, nickel foil or stainless steel foil, for example. The positive electrode collector may be coated with carbon on the surface, or it may be modified in the form of a mesh. The thickness of the positive electrode collector is preferably 5 to 40 $\mu$m, more preferably 7 to 35 $\mu$m and even more preferably 9 to 30 $\mu$m.

The basis weight per side of the positive electrode, excluding the positive electrode collector, is preferably 15 mg/cm$^2$ or greater and more preferably 17.5 mg/cm$^2$ or greater, from the viewpoint of improving the volumetric energy density of the nonaqueous secondary battery. The basis weight per side of the positive electrode, excluding the positive electrode collector, is preferably 100 mg/cm$^2$ or lower, more preferably 80 mg/cm$^2$ or lower and even more preferably 60 mg/cm$^2$ or lower. If the basis weight per side of the positive electrode excluding the positive electrode collector is limited to this range, it will be possible to provide a nonaqueous secondary battery that exhibits high output performance, even when the electrode active material layer has been designed for high volumetric energy density.

<Negative electrode>

The negative electrode 160 is composed of a negative electrode active material layer fabricated from a negative electrode mixture, and a negative electrode collector. The negative electrode 160 can function as a negative electrode for the nonaqueous secondary battery.

The negative electrode mixture comprises a negative electrode active material, and if necessary also a conductive aid and a binder.

Examples of negative electrode active materials include carbon materials including amorphous carbon (hard carbon), graphite (artificial graphite, natural graphite), pyrolytic carbon, coke, glassy carbon, fired organic polymer compounds, mesocarbon microbeads, carbon fibers, active carbon, carbon colloid, and carbon black, metal lithium, metal oxides, metal nitrides, lithium alloy, tin alloy, silicon alloy, intermetallic compounds, organic compounds, inorganic compounds, metal complexes and organic polymer compounds. One type of negative electrode active material may be used alone, or two or more may be used in combination.

The negative electrode active material used for this embodiment is preferably graphite, or a compound containing one or more elements selected from the group consisting of Ti, V, Sn, Cr, Mn, Fe, Co, Ni, Zn, Al, Si and B.

From the viewpoint of increasing the cell voltage, the negative electrode active material layer preferably contains a material that is able to store lithium ions, as the negative electrode active material, at a more electronegative potential than 0.4 V vs. Li/Li$^+$.

The negative electrode active material layer is formed by a procedure wherein a negative electrode mixture-containing slurry in which a negative electrode mixture comprising a mixture of the negative electrode active material, and a conductive aid and binder if necessary, is dispersed in a solvent (solvent removal), is coated and dried (solvent removal) onto the negative electrode collector, and pressing is carried out if necessary. The solvent used may be any publicly known one. Examples include N-methyl-2-pyrrolidone, dimethylformamide, dimethylacetamide and water.

Examples of conductive aids include carbon blacks such as acetylene black and Ketchen black; carbon fibers; and graphite. The content ratio of the conductive aid is preferably 20 parts by weight or lower and more preferably 0.1 to 10 parts by weight with respect to 100 parts by weight of the negative electrode active material.

Examples of binders include carboxymethyl cellulose, PVDF, PTFE, polyacrylic acid and fluorine rubber. Other examples include diene rubbers, such as styrene-butadiene rubber. The content ratio of the binder is preferably 10 parts by weight or lower and more preferably 0.5 to 6 parts by weight with respect to 100 parts by weight of the negative electrode active material.

The negative electrode collector is composed of a metal foil such as copper foil, nickel foil or stainless steel foil, for example. The negative electrode collector may be coated with carbon on the surface, or it may be modified in the form of a mesh. The thickness of the negative electrode collector is preferably 5 to 40 $\mu$m, more preferably 6 to 35 $\mu$m and even more preferably 7 to 30 $\mu$m.

<Separator>

The nonaqueous secondary battery 100 of this embodiment preferably comprises a separator 170 between the positive electrode 150 and negative electrode 160, from the viewpoint of providing safety functions such as preventing

short circuiting between the positive electrode 150 and negative electrode 160, or shutdown. The separator 170 preferably is an insulating thin-film with high ion permeability and excellent mechanical strength. Examples for the separator 170 include woven fabrics, nonwoven fabrics and synthetic resin microporous films, among which synthetic resin microporous films are preferred.

Examples of synthetic resin microporous films include polyolefin-based microporous films such as microporous films comprising polyethylene or polypropylene as the major component, or microporous films comprising both of these polyolefins. Examples of nonwoven fabrics include heat-resistant resin porous films made of glass, ceramic, polyolefin, polyester, polyamide, liquid crystal polyester or aramid.

The separator 170 may be composed of a single layer or multiple layers of a single type of microporous film, or it may be composed of layers of two or more microporous films. The separator 170 may also be composed of a single layer or multiple layers using a mixed resin material comprising two or more resin materials melt kneaded together.

In order to provide further functionality, inorganic particles may be added to the surface layer or interior of the separator, or it may be coated or layered with another organic layer. The separator may also be one that includes a crosslinked structure. These methods may also be combined as necessary for increased safety performance of the nonaqueous secondary battery.

Using such a separator 170 can help to produce a satisfactory input/output characteristic and a low self-discharge property as required especially for a high-output lithium ion battery. The thickness of the separator is preferably 1 μm or greater from the viewpoint of separator strength, while from the viewpoint of transparency it is preferably 500 μm or smaller, more preferably 5 μm to 30 μm and even more preferably 10 μm to 25 μm. When shorting resistance is most important, the thickness of the separator is more preferably 15 μm to 20 μm, but when high energy density is most important, it is more preferably 10 μm or greater and less than 15 μm. The porosity of the separator is preferably 30% to 90%, more preferably 35% to 80% and even more preferably 40% to 70%, from the viewpoint of following the rapid migration of lithium ions during high output. When improved output performance while maintaining safety is considered to be a priority, it is most preferably 50% to 70%, and when both shorting resistance and output performance are most important, it is most preferably at least 40% and less than 50%. From the viewpoint of balance between separator thickness and porosity, the gas permeability of the separator is preferably 1 sec/100 $cm^3$ to 400 seconds/100 $cm^3$, and more preferably 100 seconds/100 $cm^3$ to 350/100 $cm^3$. When both shorting resistance and output performance are important, the gas permeability it is most preferably 150 seconds/100 $cm^3$ to 350 seconds/100 $cm^3$, and when improvement in output performance while maintaining safety is considered to be a priority, it is most preferably 100 sec/100 $cm^3$ to less than 150 seconds/100 $cm^3$. On the other hand, when a nonaqueous electrolyte solution with low ionic conductivity is combined with a separator within this range, the rate-determining factor for the traveling speed of the lithium ions becomes the ionic conductivity of the electrolyte solution rather than the structure of the separator, and the expected input/output characteristic is less likely to be achieved. Therefore, the ionic conductivity of the nonaqueous electrolyte solution at 25°C is preferably 10 mS/cm or greater, more preferably 15 mS/cm or greater and even more preferably 20 mS/cm or greater. The thickness, gas permeability and porosity of the separator and the ionic conductivity of the nonaqueous electrolyte solution are not limited to this example, however.

<Battery exterior>

The construction of the battery exterior 110 of the nonaqueous secondary battery 100 for this embodiment may be any battery exterior such as a battery can (not shown), or a laminate film exterior body, for example. Examples of battery cans to be used include rectilinear, square cylindrical, cylindrical, elliptical, flat, coin-shaped and button-shaped metal cans composed of steel, stainless steel, aluminum or clad materials. Examples of laminate film exterior bodies include laminate films comprising a three-layer structure, such as hot molten resin/metal film/resin.

Two laminate film exterior bodies may also be stacked with their hot molten resin sides facing inward, and bent so that the hot molten resin sides are inward, and the edges sealed by heat sealing for use as an exterior body. When a laminate film exterior body is used, a positive electrode lead 130 (or a lead tab connecting positive electrode terminals and positive electrode terminals) may be connected to the positive electrode collector and a negative electrode lead 140 (or a lead tab connecting negative electrode terminals and negative electrode terminals) may be connected to the negative electrode collector. In this case, the laminate film exterior body may be sealed with the ends of the positive electrode lead 130 and negative electrode lead 140 (or the respective lead tabs connecting the positive electrode terminals and negative electrode terminals) protruding out from the exterior body.

<Method for fabricating battery>

The nonaqueous secondary battery 100 of this embodiment is fabricated by a known method using the nonaqueous electrolyte solution, the positive electrode 150 having a positive electrode active material layer on one or both sides of a current collector, the negative electrode 160 having a negative electrode active material layer on one or both

sides of a current collector, and the battery exterior 110, as well as the separator 170 as necessary.

First, a layered product is formed comprising the positive electrode 150 and negative electrode 160, and the separator 170 as necessary. For example, long sheets of the positive electrode 150 and negative electrode 160 may be wound in a layered state across a long separator situated between the positive electrode 150 and negative electrode 160, to form a layered product with a wound structure; several sheets of the positive electrode 150 and negative electrode 160 having fixed areas and shapes may be cut to obtain a positive electrode sheet and a negative electrode sheet, which are then alternately layered across separator sheets to form a layered product with a layered structure; or a long separator may be folded in a hairpin fashion, and positive electrode sheets and negative electrode sheets inserted between the hairpin-folded separator to form a layered product with a layered structure.

The layered product may then be housed in the battery exterior 110 (battery case), the electrolyte solution of this embodiment may be filled inside the battery case, immersing the layered product in the electrolyte solution, and it may then be sealed to fabricate a nonaqueous secondary battery for this embodiment. Alternatively, the electrolyte solution may be impregnated into a substrate composed of a polymer material, to form a gelled electrolyte membrane in advance, and then the positive electrode 150, negative electrode 160 and electrolyte membrane, and the separator 170 as necessary, in the form of sheets, may be used to form a layered product with a layered structure, after which they may be housed in the battery exterior 110 to fabricate the nonaqueous secondary battery 100.

When the placement of the electrodes is designed so that overlapping portions exist between the outer perimeter edges of the negative electrode active material layer and the outer perimeter edges of the positive electrode active material layer, or so that sections exist in the non-facing part of the negative electrode active material layer where the width is too short, dislocation of the electrodes may take place during assembly of the battery, potentially leading to a lower charge-discharge cycle characteristic of the nonaqueous secondary battery. Therefore, the electrode structure used in the nonaqueous secondary battery preferably has the locations of the electrodes anchored with tape such as polyimide tape, polyphenylene sulfide tape or PP tape, or an adhesive.

Because of the high ionic conductivity of acetonitrile for this embodiment, the lithium ions released from the positive electrode during initial charge of the nonaqueous secondary battery can potentially diffuse throughout the entire negative electrode. In a nonaqueous secondary battery it is common for the area of the negative electrode active material layer to be made larger than that of the positive electrode active material layer. However, when lithium ions diffuse and become stored even in the sections of the negative electrode active material layer that are not facing the positive electrode active material layer, these lithium ions will reside in the negative electrode without being released during initial discharge. The contribution of the non-released lithium ions therefore constitutes irreversible capacity. For this reason, a nonaqueous secondary battery using a nonaqueous electrolyte solution that contains acetonitrile often has lowered initial charge-discharge efficiency.

On the other hand, when the area of the positive electrode active material layer is larger than that of the negative electrode active material layer, or when both are the same, current tends to become concentrated at the edge sections of the negative electrode active material layer during charge, resulting in formation of lithium dendrites.

There is no particular limitation on the ratio of the area of the entire negative electrode active material layer with respect to the area of the part where the positive electrode active material layer and negative electrode active material layer are facing, but for the reasons explained above, it is preferably greater than 1.0 and less than 1.1, more preferably greater than 1.002 and less than 1.09, even more preferably greater than 1.005 and less than 1.08, and most preferably greater than 1.01 and less than 1.08. In a nonaqueous secondary battery using a nonaqueous electrolyte solution that contains acetonitrile, the initial charge-discharge efficiency can be improved by lowering the ratio of the area of the entire negative electrode active material layer with respect to the area of the part where the positive electrode active material layer and negative electrode active material layer are facing.

Lowering the ratio of the area of the entire negative electrode active material layer with respect to the area of the part where the positive electrode active material layer and negative electrode active material layer are facing, means limiting the proportion of the area of the locations of the negative electrode active material layer that are not facing the positive electrode active material layer. This can maximally reduce the amount of lithium ions stored at locations of the negative electrode active material layer that are not facing the positive electrode active material layer (that is, the amount of lithium ions that are not released from the negative electrode during initial discharge, constituting irreversible capacity), among the lithium ions released from the positive electrode during initial charge. If the design thus limits the range for the ratio of the area of the entire negative electrode active material layer with respect to the area of the locations where the positive electrode active material layer and negative electrode active material layer are facing, then it will be possible to achieve an increased load characteristic for the battery by using acetonitrile, while increasing the initial charge-discharge efficiency of the battery and also inhibiting formation of lithium dendrites.

The nonaqueous secondary battery 100 of this embodiment can function as a battery by initial charge, but it is stabilized by partial decomposition of the electrolyte solution during initial charge. There are no particular restrictions on the method of initial charge, but preferably the initial charge is carried out at 0.001 to 0.3 C, more preferably 0.002 to 0.25 C and even more preferably 0.003 to 0.2 C. Carrying out the initial charge by constant-voltage charge also provides

a desirable result. By setting a larger voltage range for electrochemical reaction of the lithium salt, a stable, firm negative electrode SEI is formed on the electrode surface and internal resistance increase is inhibited, while the reaction product does not become firmly immobilized only on the negative electrode 160, but rather a satisfactory effect is also provided for the elements other than the negative electrode 160, such as the positive electrode 150 and separator 170. It is therefore highly effective to carry out the initial charge in consideration of electrochemical reaction of the lithium salt dissolved in the nonaqueous electrolyte solution.

The nonaqueous secondary battery 100 of this embodiment may consist of several nonaqueous secondary batteries 100 connected in series or parallel for use as a battery pack. From the viewpoint of controlling the charge-discharge state of the battery pack, the working voltage range per battery is preferably 1.5 to 4.0 V and most preferably 2.0 to 3.8 V, when a positive electrode active material represented by (Xba) is used as the positive electrode.

When a positive electrode active material represented by formula ($a^t$) is used, the working voltage range per battery is preferably 2 to 5 V, more preferably 2.5 to 5 V and most preferably 2.75 V to 5 V.

<Second embodiment>

The nonaqueous electrolyte solution of the second embodiment, and a nonaqueous secondary battery that includes it, will now be described.

By using a nonaqueous secondary battery according to this embodiment it is possible, firstly, to provide a nonaqueous electrolyte solution with reduced degradation reaction in the nonaqueous secondary battery and reduced self-discharge at high temperatures above 60°C, as a result of reduced decomposition reaction of the nonaqueous electrolyte solution components by active oxygen species generated during the electrochemical reaction, resulting in improved output characteristics and cycle performance in a wide range of temperatures, as well as a nonaqueous secondary battery comprising the solution. It is also possible, secondly, to provide a nonaqueous electrolyte solution wherein the mixing ratio of lithium (Li) salts is controlled to avoid lack of negative electrode SEI formation and corrosion of the aluminum (Al) current collector while also inhibiting degradation reaction in the nonaqueous secondary battery caused by heat degradation of Li salts, and wherein it is possible to improve the output characteristic of the nonaqueous secondary battery in a wide range of temperatures, as well as the long-term durability and cycle performance at high temperatures above 60°C, as well as a nonaqueous secondary battery comprising the solution.

For this embodiment, each of the elements used to form the nonaqueous secondary battery may be the respective elements explained for the first embodiment. The preferred mode for this embodiment, and the function and effect based on that mode, is as explained for the first embodiment.

<2. Electrolyte solution>

The electrolyte solution of the second embodiment is the same as for the first embodiment, except for the lithium salt.

<2-1. Nonaqueous solvent>

The nonaqueous solvent of the second embodiment is the same as that of the first embodiment.

<2-2. Lithium salt>

The nonaqueous electrolyte solution of the second embodiment contains $LiPF_6$ and a lithium-containing imide salt as lithium salts. A lithium-containing imide salt is a lithium salt represented by $LiN(SO_2C_mF_{2m+1})_2$ [where m is an integer of 0 to 8], and specifically, it preferably includes at least one of $LiN(SO_2F)_2$ and $LiN(SO_2CF_3)_2$, and more preferably includes $LiN(SO_2F)_2$. In the relevant technical field, $LiN(SO_2F)_2$ is known as lithium bis(fluorosulfonyl)imide, and is sometimes abbreviated as LiFSI. The lithium salt may also include a lithium-containing imide salt other than these lithium-containing imide salts.

The content of the $LiPF_6$ in the nonaqueous electrolyte solution of this embodiment is 0.01 mol or greater and less than 0.1 mol, preferably 0.020 mol or greater, even more preferably 0.030 mol or greater and yet more preferably 0.045 mol or greater, to 1 L of the nonaqueous solvent. If the $LiPF_6$ content is within this range it will be possible to ensure an amount of HF necessary for aluminum passivation and negative electrode SEI formation. When the $LiPF_6$ content in the nonaqueous electrolyte solution is low, there is a risk of corrosion reaction of the aluminum of the current collector by the lithium-containing imide salt and reductive decomposition of the electrolyte solution component due to insufficient negative electrode SEI formation, but HF is also generated from $PF_6$ anion as hydrogen is removed from the $\alpha$-position of acetonitrile, thereby accelerating aluminum passivation and negative electrode SEI formation. Even if the $LiPF_6$ content is reduced, therefore, it is possible to ensure an amount of HF necessary for aluminum passivation and

negative electrode SEI formation. The generated HF is thought to be consumed by aluminum passivation and negative electrode SEI formation, making it possible to minimize loss of battery performance due to corrosion reaction and electrolyte solution degradation by acid components.

The $LiPF_6$ content is preferably 0.090 mol or lower, more preferably 0.080 mol or lower and even more preferably 0.070 mol or lower, with respect to 1 L of the nonaqueous solvent. $LiPF_6$ is known to thermally decompose at high temperature, forming an acid component. This tendency is particularly notable in environments with temperatures above 60°C, and even more notable in high-temperature environments above 80°C. The tendency is also notable when the exposure time to high temperature is 4 hours or longer, more notable when it is 24 hours or longer, even more notable when it is 10 days or longer, yet more notable when it is 20 days or longer, and even yet more notable when it is 30 days or longer. If the $LiPF_6$ content is within the range specified above, it will be possible to inhibit generation of acid component by thermal decomposition reaction of $LiPF_6$ even after exposure for 4 hours or longer to a temperature above 60°C. It will also be possible to reduce the amount of HF generated from $PF_6$ anion by removal of hydrogen from the $\alpha$-position of acetonitrile. If the amount of HF generated in the electrolyte solution is high, then the metal component will elute from the battery element, not only causing corrosion of the elements but also generating complex cations from the eluted metal ion and acetonitrile, and accelerating degradation of the battery. This tendency is particularly notable in environments with temperatures above 60°C, and even more notable in high-temperature environments above 80°C. The tendency is also notable when the exposure time to high temperature is 4 hours or longer, more notable when it is 24 hours or longer, even more notable when it is 10 days or longer, yet more notable when it is 20 days or longer, and even yet more notable when it is 30 days or longer. If the $LiPF_6$ content is within the range specified above, however, it will be possible to reduce the amount of HF generated from $PF_6$ anion by removal of hydrogen from the $\alpha$-position of acetonitrile and to inhibit progressive degradation, even after exposure for 4 hours or longer at a temperature above 60°C. This can help maintain a minimal level of reduction in battery performance due to corrosion of the positive electrode active material layer and positive electrode collector, or degradation reaction of the electrolyte solution.

Since the saturation concentration of the lithium-containing imide salt in acetonitrile is higher than the saturation concentration of $LiPF_6$, the molar concentrations are preferred such that $LiPF_6 \leq$ lithium-containing imide salt. From the viewpoint of inhibiting association and precipitation of the lithium salt and acetonitrile at low temperature, the molar ratio of the lithium-containing imide salt with respect to the $LiPF_6$ is greater than 10, preferably 15 or greater, more preferably 20 or greater and even more preferably 25 or greater. The lithium-containing imide salt content is also preferably 0.5 mol to 3 mol with respect to 1 L of nonaqueous solvent, from the viewpoint of the ion supply rate. With an acetonitrile-containing nonaqueous electrolyte solution including either or both $LiN(SO_2F)_2$ and $LiN(SO_2CF_3)_2$, it is possible to effectively inhibit reduction in the ion conductivity in the low-temperature region, such as -10°C or -40°C, and to obtain excellent low-temperature characteristics. By controlling the contents of each of the components in this manner it is possible to more effectively inhibit resistance increase during high-temperature heating.

The lithium salt used in the nonaqueous electrolyte solution of this embodiment may also include a fluorine-containing inorganic lithium salt other than $LiPF_6$, and for example, it may include a fluorine-containing inorganic lithium salt such as $LiBF_4$, $LiAsF_6$, $Li_2SiF_6$, $LiSbF_6$ or $Li_2B_{12}F_bH_{12-b}$ [where b is an integer of 0 to 3]. The term "inorganic lithium salt" means an acetonitrile-soluble lithium salt that includes no carbon atoms in the anion. The term "fluorine-containing inorganic lithium salt" means an acetonitrile-soluble lithium salt that includes no carbon atoms in the anion and includes fluorine in the anion. The fluorine-containing inorganic lithium salt forms a passivation film on the surface of the aluminum foil as the positive electrode collector, which is excellent for inhibiting corrosion of the positive electrode collector. Such fluorine-containing inorganic lithium salts may be used alone, or two or more may be used in combination. Preferred fluorine-containing inorganic lithium salts are compounds that are compound salts of LiF and Lewis acids, among which the use of fluorine-containing inorganic lithium salts with phosphorus atoms is more preferred because they readily release free fluorine. The fluorine-containing inorganic lithium salt is preferably a fluorine-containing inorganic lithium salt, with a boron atom preferred because it can more easily trap excess free acid component that can potentially lead to battery degradation, and therefore $LiBF_4$ is especially preferred.

The content of the fluorine-containing inorganic lithium salt in the lithium salt used in the nonaqueous electrolyte solution of this embodiment is preferably 0.01 mol or greater, more preferably 0.1 mol or greater and even more preferably 0.25 mol or greater, to 1 L of the nonaqueous solvent. If the fluorine-containing inorganic lithium salt content is within this range, the ionic conductivity will tend to increase, allowing a high output characteristic to be exhibited. The content is preferably lower than 2.8 mol, more preferably lower than 1.5 mol and even more preferably lower than 1 mol, with respect to 1 L of the nonaqueous solvent. If the fluorine-containing inorganic lithium salt content is within this range, the ionic conductivity will tend to increase, allowing a high output characteristic to be exhibited by the battery, while also helping to inhibit decrease in ionic conductivity due to increased viscosity at low temperature, and will tend to result in even more satisfactory high temperature cycle characteristics and other battery characteristics, while maintaining the excellent performance of the nonaqueous electrolyte solution. The nonaqueous electrolyte solution of this embodiment may further include an organic lithium salt.

Organic lithium salts include organic lithium salts having an oxalic acid structure. Specific examples of organic

lithium salts with an oxalic acid structure include organic lithium salts represented by $LiB(C_2O_4)_2$, $LiBF_2(C_2O_4)$, $LiPF_4(C_2O_4)$ and $LiPF_2(C_2O_4)_2$, among which one or more lithium salts represented by $LiB(C_2O_4)_2$ and $LiBF_2(C_2O_4)$ are preferred. More preferably, one or more of these is used together with a fluorine-containing inorganic lithium salt. The organic lithium salt with an oxalic acid structure may also be added to the negative electrode active material layer, in addition to being added to the nonaqueous electrolyte solution.

From the viewpoint of more satisfactorily ensuring that an effect is exhibited, the amount of organic lithium salt with an oxalic acid structure added to the nonaqueous electrolyte solution is preferably 0.005 mol or greater, more preferably 0.02 mol or greater and even more preferably 0.05 mol or greater, per 1 L of nonaqueous solvent of the nonaqueous electrolyte solution. However, excess addition of an organic lithium salt with an oxalic acid structure to the nonaqueous electrolyte solution can potentially lead to precipitation from the nonaqueous electrolyte solution. Therefore, the amount of organic lithium salt with an oxalic acid structure added to the nonaqueous electrolyte solution is preferably less than 1.0 mol, more preferably less than 0.5 mol and even more preferably less than 0.2 mol, to 1 L of the nonaqueous solvent of the nonaqueous electrolyte solution.

Organic lithium salts with an oxalic acid structure are known to be poorly soluble in low-polarity organic solvents, and especially chain carbonates. An organic lithium salt with an oxalic acid structure sometimes contains trace amounts of lithium oxalate, and when mixed in with a nonaqueous electrolyte solution, it reacts with trace amounts of water in the other starting materials to result in new white precipitation of lithium oxalate. The lithium oxalate content in the nonaqueous electrolyte solution of this embodiment is therefore preferably 0 to 500 ppm.

Lithium salts commonly used for nonaqueous secondary batteries may also be supplementarily added as lithium salts for this embodiment, in addition to those mentioned above. Specific examples of other lithium salts include inorganic lithium salts without fluorine in the anions, such as $LiClO_4$, $LiAlO_4$, $LiAlCl_4$, $LiB_{10}Cl_{10}$ and lithium chloroborane; organic lithium salts such as $LiCF_3SO_3$, $LiCF_3CO_2$, $Li_2C_2F_4(SO_3)_2$, $LiC(CF_3SO_2)_3$, $LiC_nF_{(2n+1)}SO_3$ ($n \geq 2$), lower aliphatic carboxylic acid Li salts, Li tetraphenylborate and $LiB(C_3O_4H_2)_2$; organic lithium salts represented by $LiPF_n(C_pF_{2p+1})_{6-n}$ (e.g. $LiPF_5(CF_3)$) [where n is an integer of 1 to 5 and p is an integer of 1 to 8]; organic lithium salts represented by $LiBF_q(C_sF_{2s+1})_{4-q}$ (e.g. $LiBF_3(CF_3)$) [where q is an integer of 1 to 3 and s is an integer of 1 to 8];

multivalent anion-bonded lithium salts; and
organic lithium salts represented by the following formula (a):

$$LiC(SO_2R^A)(SO_2R^B)(SO_2R^C) \qquad (a)$$

{where $R^A$, $R^B$ and $R^C$ may be the same or different and each represents a perfluoroalkyl group of 1 to 8 carbon atoms}, the following formula (b):

$$LiN(SO_2OR^D)(SO_2OR^E) \qquad (b)$$

{where $R^D$ and $R^E$ may be the same or different and each represents a perfluoroalkyl group of 1 to 8 carbon atoms}, and the following formula (c):

$$LiN(SO_2R^F)(SO_2OR^G) \qquad (c)$$

{where $R^F$ and $R^G$ may be the same or different and each represents a perfluoroalkyl group of 1 to 8 carbon atoms},

and any one or two or more of these may be used.

<2-3. Electrode-protecting additive>

The electrode-protecting additive of the second embodiment is the same as for the first embodiment.

<2-4. Other optional additives>

Optional additives for the second embodiment are the same as for the first embodiment.

<2-5. Condensation polycyclic heterocyclic ring compound>

The condensation polycyclic heterocyclic ring compound of the second embodiment is the same as for the first embodiment.

<3. Positive electrode>

The positive electrode of the second embodiment is the same as for the first embodiment and is not particularly restricted, but it is particularly preferred to use a lithium-phosphorus metal oxide having an olivine crystal structure that includes an iron (Fe) atom, and it is more preferred to use a lithium-phosphorus metal oxide having an olivine structure represented by the following formula (Xba):

$$Li_wM^{II}PO_4 \qquad (Xba)$$

{where $M^{II}$ represents one or more transition metal elements, including at least one transition metal element that contains Fe, and the value of w is determined by the charge-discharge state of the battery and represents a value of 0.05 to 1.10}.
When a positive electrode active material represented by formula ($a^t$) is used, there is a risk of corrosion reaction of the Al current collector by the lithium-containing imide salt in voltage ranges exceeding 4.0 V. The extent to which $LiPF_6$ can be reduced has therefore been limited, since it is necessary for formation of a passivation film on the Al current collector. On the other hand, a lithium-phosphorus metal oxide with an olivine structure represented by formula (Xba) has a low nominal voltage of about 3.2 V, making it possible to avoid the voltage range in which corrosion reaction of the Al current collector takes place, so that the $LiPF_6$ can be further reduced. It is known that $LiPF_6$ decomposes at high temperature producing strong acid, and therefore reducing it can minimize degradation of batteries.
For structural stabilization, these lithium-containing metal oxides may be ones having a portion of the transition metal element replaced with Al, Mg, or another transition metal element, or having the metal elements added at the grain boundaries, or having some of the oxygen atoms replaced with fluorine atoms, or having another positive electrode active material covering at least part of the positive electrode active material surface.
Examples of positive electrode active materials include phosphoric acid metal oxides containing lithium and a transition metal element, and silicic acid metal oxides containing lithium and a transition metal element. From the viewpoint of obtaining higher voltage, a lithium-containing metal oxide is preferably a phosphoric acid metal oxide containing lithium and at least one transition metal element selected from the group consisting of Co, Ni, Mn, Fe, Cu, Zn, Cr, V and Ti, and from the viewpoint of the lithium-phosphorus metal oxide represented by formula (Xba) above, it is more preferably a phosphoric acid metal oxide containing Li and Fe.
Lithium-phosphorus metal oxides to be used that are different from lithium-phosphorus metal oxides represented by formula (Xba) include compounds represented by the following formula (Xa):

$$Li_vM^ID_2 \qquad (Xa)$$

{where D represents a chalcogen element, $M^I$ represents one or more transition metal elements that include at least one type of transition metal element, and the value of v is determined by the charge-discharge state of the battery and represents a value of 0.05 to 1.10}.

<4. Negative electrode>

The negative electrode of the second embodiment is the same as that of the first embodiment.

<5. Separator>

The separator of the second embodiment is the same as that of the first embodiment.

<6. Battery exterior>

The battery exterior of the second embodiment is the same as that of the first embodiment.

<7. Method for fabricating battery>

The method for fabricating the battery of the second embodiment is the same as that of the first embodiment.

<Third embodiment>

The nonaqueous electrolyte solution of the third embodiment, and a nonaqueous secondary battery that includes it, will now be described. By using a nonaqueous secondary battery of this embodiment, it is possible to provide a nonaqueous electrolyte solution wherein the mixing ratio of lithium (Li) salts is controlled to avoid lack of negative

electrode SEI formation and corrosion of the aluminum (Al) current collector while also inhibiting degradation reaction in the nonaqueous secondary battery caused by heat degradation of Li salts, and wherein it is possible to improve the output characteristic of the nonaqueous secondary battery in a wide range of temperatures, and the long-term durability and cycle performance at high temperatures above 60°C, as well as a nonaqueous secondary battery comprising the solution.

For the third embodiment, each of the elements used to form the nonaqueous secondary battery may be the respective elements explained for the first and second embodiments. The preferred mode for this embodiment, and the function and effect based on that mode, is as explained for the first and second embodiments.

<2. Electrolyte solution>

<2-1. Nonaqueous solvent>

The nonaqueous solvent of the third embodiment is the same as that of the first embodiment.

In an electrolyte solution containing acetonitrile as the nonaqueous solvent, the flash point of the nonaqueous electrolyte solution is lowered by addition of the acetonitrile that has a low flash point. Therefore, among the nonaqueous solvents of the first embodiment, it is preferred to add two or more different high-flash-point solvents with flash points of 50°C or higher at 1 atmospheric pressure. The flash point of the high-flash-point solvent is preferably 60°C or higher and more preferably 70°C or higher.

From the viewpoint of increasing the flash point without impairing the excellent low-temperature characteristics of the acetonitrile, the melting point of the high-flash-point solvent is preferably -20°C or lower, more preferably -30°C or lower and even more preferably -40°C or lower.

Examples of high-flash-point solvents satisfying this condition include γ-butyrolactone, α-methyl-γ-butyrolactone and diisobutyl carbonate.

The total content of the high-flash-point solvent is preferably 10% or higher, more preferably 15% or higher and even more preferably 20% or higher. If the total content of the high-flash-point solvent is restricted to this range it will be possible to more effectively increase the flash point of the nonaqueous electrolyte solution. The total content of the high-flash-point solvent is also preferably 80% or lower, more preferably 70% or lower and even more preferably 60% or lower. If the total content of the high-flash-point solvent is restricted to this range it will be possible to adequately increase the flash point without impairing the excellent output characteristics of the acetonitrile.

The flash point of the nonaqueous electrolyte solution at 1 atmospheric pressure is preferably 21°C or higher, more preferably 22°C or higher and even more preferably 24°C or higher, from the viewpoint of ensuring at least the same level of safety as a commercially available nonaqueous electrolyte solution.

<2-2. Lithium salt>

The lithium salt of the third embodiment is the same as for the second embodiment.

<2-3. Electrode-protecting additive>

The electrode-protecting additive of the third embodiment is the same as for the first embodiment.

<2-4. Other optional additives>

Optional additives for the third embodiment are the same as for the first embodiment.

<3. Positive electrode>

The positive electrode of the third embodiment is the same as that of the second embodiment.

<4. Negative electrode>

The negative electrode of the third embodiment is the same as that of the first embodiment.

<5. Separator>

The separator of the third embodiment is the same as that of the first embodiment.

<6. Battery exterior>

The battery exterior of the third embodiment is the same as that of the first embodiment.

<7. Method for fabricating battery>

The method for fabricating the battery of the third embodiment is the same as that of the first embodiment.

The manner of carrying out the present invention was explained above, but the invention is not limited to the embodiments described above.

EXAMPLES

The present invention will now be explained in greater detail by examples. However, it is to be understood that the invention is not limited to these examples.

(1) Preparation of nonaqueous electrolyte solution

Each of the nonaqueous solvents, acid anhydrides and additives were mixed under an inert atmosphere to their prescribed concentrations, and the lithium salts were added to their prescribed concentrations, to prepare non-aqueous electrolyte solutions (S1) to (S45).

(2) Measurement of ionic conductivity of nonaqueous electrolyte solutions

The nonaqueous electrolyte solutions obtained in this manner were each measured for ionic conductivity.

The ionic conductivity measurement was conducted in a 25°C environment, measuring the electrical conductivity of the nonaqueous electrolyte solution using a conductivity meter (X-Series benchtop water quality meter: CM-41X).

The nonaqueous electrolyte solution compositions and the ionic conductivity measurement results are shown in Table 1.

The names of the nonaqueous solvents, lithium salts and additives in Table 1 are as follows. The parts by weight of the additives in Table 1 are parts by weight with respect to 100 parts by weight of each nonaqueous electrolyte solution without the additives.

(Nonaqueous solvents)

AcN: Acetonitrile
DEC: Diethyl carbonate
EMC: Ethylmethyl carbonate
DMC: Dimethyl carbonate
DFA: 2,2-Difluoroethyl acetate
DIBC: Diisobutyl carbonate
GBL: γ-Butyrolactone
MBL: α-Methyl-γ-butyrolactone
EC: Ethylene carbonate
ES: Ethylene sulfite
VC: Vinylene carbonate

(Lithium salts)

$LiPF_6$: Lithium hexafluorophosphate
LiFSI: Lithium bis(fluorosulfonyl)imide

(Additives: Others)

SAH: Succinic anhydride
TEGS: Triethoxy(3-glycidyloxypropyl)silane
MPPZ: 3-Methyl-1-phenyl-5-pyrazolone
EPZ: 1-Ethyl-1H-pyrazole
DPP: 2,6-Di(1-pyrazolyl)pyridine

PPZ: 3-Phenyl-1H-pyrazole
PZP: 2-(1H-Pyrazol-3-yl)pyridine
DPPZ: 3,5-Diphenylpyrazole
INZ: Indazole
PD: Pyridine
MBTA: 1-Methylbenzotriazole
CAF: 1,3,7-Trimethylxanthine(caffeine)

[Table 1-1]

| Electrolyte solution No. | Lithium salt (mol/l L solvent) | | Nonaqueous solvent (parts by volume) | | | | | | | | | | | Additive (parts by weight) | | Nitrogen-containing heterocyclic ring compound | | Physical property value |
|---|---|---|---|---|---|---|---|---|---|---|---|---|---|---|---|---|---|---|
| | LiPF6 | LiFSI | AcN | DEC | EMC | DMC | DIBC | DFA | GBL | MBL | EC | ES | VC | SAH | TEGS | Type | Addition amount (parts by weight) | Ionic conductivity [mS/cm] |
| S1 | 0.300 | 1.000 | 34.5 | | | | | 20.0 | 22.0 | | 21.0 | | 2.5 | | | CAF | 0.25 | 19.3 |
| S2 | 0.300 | 1.000 | 34.5 | | | | 22.0 | 20.0 | | | 21.0 | | 2.5 | | | CAF | 0.25 | 20.1 |
| S3 | 0.300 | 1.000 | 38.5 | | | | 23.0 | | 32.0 | | | 4.0 | 2.5 | | | CAF | 0.25 | 19.4 |
| S4 | 0.300 | 1.000 | 49.0 | 28.0 | | | | | | | 21.0 | | 2.0 | 0.2 | | CAF | 0.25 | 21.6 |
| S5 | 0.360 | 0.940 | 43.5 | | | | | | | 5.9 | 49.6 | | 1.0 | | 0.58 | CAF | 0.12 | 20.7 |
| S6 | 0.300 | 1.000 | 49.0 | 28.0 | | | | | | | 21.0 | | 2.0 | | | CAF | 0.10 | 21.7 |
| S7 | 0.300 | 1.000 | 49.0 | 28.0 | | | | | | | 21.0 | | 2.0 | 0.2 | | CAF | 0.10 | 21.7 |
| S8 | 0.300 | 1.000 | 34.5 | | 43.0 | | | | | | 20.0 | | 2.5 | | | CAF | 0.25 | 20.1 |
| S9 | 0.300 | 1.000 | 10.0 | 66.5 | | | | | | | 21.0 | | 2.5 | | | CAF | 0.10 | 10.0 |

[Table 1-2]

| Electrolyte solution No. | Lithium salt (mol/l L solvent) | | Nonaqueous solvent (parts by volume) | | | | | | | | | | | Additive (parts by weight) | | Nitrogen-containing heterocyclic ring compound | | Physical property value |
|---|---|---|---|---|---|---|---|---|---|---|---|---|---|---|---|---|---|---|
| | LiPF6 | LiFSI | AcN | DEC | EMC | DMC | DIBC | DFA | GBL | MBL | EC | ES | VC | SAH | TEGS | Type | Addition amount (parts by weight) | Ionic conductivity [mS/cm] |
| S10 | 0.050 | 1.250 | 49.0 | 28.0 | | | | | | | 21.0 | | 2.0 | | | CAF | 0.25 | 21.6 |
| Sil | 0.050 | 1.250 | 38.5 | | | | 23.0 | | 32.0 | | | 4.0 | 2.5 | | | CAF | 0.25 | 19.4 |
| S12 | 0.050 | 1.250 | 34.5 | | | | | 20.0 | 22.0 | | 21.0 | | 2.5 | | | CAF | 0.25 | 19.7 |
| S13 | 0.050 | 1.250 | 34.5 | | | | 22.0 | 20.0 | | | 21.0 | | 2.5 | | | CAF | 0.25 | 20.0 |

[Table 1-3]

| Electrolyte solution No. | Lithium salt (mol/l L solvent) | | Nonaqueous solvent (parts by volume) | | | | | | | | | | | Additive (parts by weight) | | Nitrogen-containing heterocyclic ring compound | | Physical property value |
|---|---|---|---|---|---|---|---|---|---|---|---|---|---|---|---|---|---|---|
| | LiPF6 | LiFSI | AcN | DEC | EMC | DMC | DIBC | DFA | GBL | MBL | EC | ES | VC | SAH | TEGS | Type | Addition amount (parts by weight) | Ionic conductivity [mS/cm] |
| S14 | 0.050 | 1.250 | 38.5 | | | | 23.0 | | 32.0 | | | 4.0 | 2.5 | | | | | 19.3 |
| S15 | 0.075 | 1.225 | 49.0 | 28.0 | | | | | | | 21.0 | | 2.0 | 0.2 | | MBTA | | 21.7 |
| S16 | 0.050 | 1.250 | 49.0 | 28.0 | | | | | | | 21.0 | | 2.0 | 0.2 | | MBTA | | 21.7 |
| S17 | 0.025 | 1.275 | 49.0 | 28.0 | | | | | | | 21.0 | | 2.0 | 0.2 | | MBTA | | 21.7 |
| S18 | 0.050 | 1.250 | 49.0 | 28.0 | | | | | | | 21.0 | | 2.0 | 0.2 | | | | 21.7 |
| S19 | 0.025 | 1.275 | 49.0 | 28.0 | | | | | | | 21.0 | | 2.0 | 0.2 | | | | 21.7 |
| S20 | 0.075 | 1.225 | 49.0 | 28.0 | | | | | | | 21.0 | | 2.0 | 0.2 | | | | 21.7 |
| S21 | 0.050 | 1.250 | 49.0 | 28.0 | | | | | | | 21.0 | | 2.0 | | | MBTA | 0.25 | 21.8 |
| S22 | 0.050 | 1.250 | 49.0 | 28.0 | | | | | | | 21.0 | | 2.0 | | | | | 21.7 |

(Electrolyte solutions S14 to S22 are not in accordance with the claimed invention)

[Table 1-4]

| Electrolyte solution No. | Lithium salt (mol/l L solvent) | | Nonaqueous solvent (parts by volume) | | | | | | | | | | | Additive (parts by weight) | | Nitrogen-containing heterocyclic ring compound | | Physical property value |
|---|---|---|---|---|---|---|---|---|---|---|---|---|---|---|---|---|---|---|
| | LiPF6 | LiFSI | AcN | DEC | EMC | DMC | DIBC | DFA | GBL | MBL | EC | ES | VC | SAH | TEGS | Type | Addition amount (parts by weight) | Ionic conductivity [mS/cm] |
| S23 | 1.000 | | | | 69.0 | | | | | | 29.0 | | 2.0 | | | CAF | 0.25 | 10.5 |
| S24 | 1.000 | | | | 69.0 | | | | | | 29.0 | | 2.0 | | | | | 10.3 |
| S25 | 1.200 | | | | 29.0 | 37.0 | | | | | 34.0 | | | | | | | 12.7 |
| S26 | 0.300 | 1.000 | 49.0 | 28.0 | | | | | | | 21.0 | | 2.0 | 0.2 | | PZP | 0.10 | 21.7 |
| S27 | 0.300 | 1.000 | 49.0 | 28.0 | | | | | | | 21.0 | | 2.0 | 0.2 | | DPP | 0.10 | 21.7 |
| S28 | 0.300 | 1.000 | 49.0 | 28.0 | | | | | | | 21.0 | | 2.0 | 0.2 | | DPPZ | 0.10 | 21.7 |
| S29 | 0.300 | 1.000 | 49.0 | 28.0 | | | | | | | 21.0 | | 2.0 | 0.2 | | PPZ | 0.10 | 21.7 |
| S30 | 0.300 | 1.000 | 49.0 | 28.0 | | | | | | | 21.0 | | 2.0 | 0.2 | | EPZ | 0.10 | 21.7 |
| S31 | 0.300 | 1.000 | 49.0 | 28.0 | | | | | | | 21.0 | | 2.0 | 0.2 | | INZ | 0.10 | 21.7 |
| S32 | 0.300 | 1.000 | 49.0 | 28.0 | | | | | | | 21.0 | | 2.0 | 0.2 | | MBTA | 0.25 | 21.6 |
| S33 | 0.300 | 1.000 | 49.0 | 28.0 | | | | | | | 21.0 | | 2.0 | | | MBTA | 0.50 | 21.4 |
| (Electrolyte solutions S23 to S33 are not in accordance with the claimed invention) | | | | | | | | | | | | | | | | | | |

[Table 1-5]

| Electrolyte solution No. | Lithium salt (mol/l L solvent) | | Nonaqueous solvent (parts by volume) | | | | | | | | | | | Additive (parts by weight) | | Nitrogen-containing heterocyclic ring compound | | Physical property value |
|---|---|---|---|---|---|---|---|---|---|---|---|---|---|---|---|---|---|---|
| | LiPF6 | LiFSI | AcN | DEC | EMC | DMC | DIBC | DFA | GBL | MBL | EC | ES | VC | SAH | TEGS | Type | Addition amount (parts by weight) | Ionic conductivity [mS/cm] |
| S34 | 0.300 | 1.000 | 48.5 | | 28.0 | | | | | | 21.0 | | 2.5 | | | PD | 0.12 | 23.0 |
| S35 | 0.300 | 1.000 | 48.5 | | 28.0 | | | | | | 21.0 | | 2.5 | | | PD | 0.04 | 23.1 |
| S36 | 0.300 | 1.000 | 49.0 | 28.0 | | | | | | | 21.0 | | 2.0 | 0.2 | | | | 21.6 |
| S37 | 0.300 | 1.000 | 49.0 | 28.0 | | | | | | | 21.0 | | 2.0 | 0.2 | | MPPZ | 0.10 | 21.7 |
| S38 | 0.300 | 1.000 | 49.0 | 28.0 | | | | | | | 21.0 | | 2.0 | 0.2 | | MPPZ | 0.25 | 21.6 |
| S39 | 0.300 | 1.000 | 49.0 | 28.0 | | | | | | | 21.0 | | 2.0 | 0.2 | | MPPZ | 0.50 | 21.4 |
| S40 | 0.300 | 1.000 | 49.0 | 28.0 | | | | | | | 21.0 | | 2.0 | 0.2 | | MBTA | 0.10 | 21.7 |
| S41 | 0.300 | 1.000 | 10.0 | 66.5 | | | | | | | 21.0 | | 2.5 | | | | | 7.8 |
| S42 | 0.300 | 1.000 | 34.5 | | | | | 20.0 | 22.0 | | 21.0 | | 2.5 | | | | | 19.4 |
| S43 | 0.050 | 1.250 | | | 69.0 | | | | | | 29.0 | | 2.0 | | | | | 10.4 |
| S44 | | 1.300 | 49.0 | 28.0 | | | | | | | 21.0 | | 2.0 | 0.2 | | | | 21.1 |
| S45 | | 1.300 | 49.0 | 28.0 | | | | | | | 21.0 | | 2.0 | 0.2 | | | | 21.1 |

(Electrolyte solutions S34 to S45 are not in accordance with the claimed invention)

(3) Fabrication of coin-type nonaqueous secondary battery

(3-1) Fabrication of positive electrode

(3-1-1) Fabrication of positive electrode (P1)

A positive electrode mixture was obtained by mixing (A) iron phosphate lithium (LiFePOq) having an olivine-type structure as the positive electrode active material, (B) carbon black powder as a conductive aid and polyvinylidene fluoride (PVDF) as a binder, in a weight ratio of 84:10:6.

After then adding N-methyl-2-pyrrolidone as a solvent to the obtained positive electrode mixture to a solid content of 68 weight%, mixing was continued to prepare a positive electrode mixture-containing slurry. One side of an aluminum foil with a thickness of 15 μm and a width of 280 mm, serving as the positive electrode collector, was coated with the positive electrode mixture-containing slurry using a triple roll-type transfer coater, to a coating pattern with a coating width of 240 to 250 mm, a coating length of 125 mm and a non-coating length of 20 mm, while adjusting the basis weight, and the solvent was dried off with a hot air drying oven. The obtained electrode roll was trimmed on both sides and dried under reduced pressure at 130°C for 8 hours. It was then rolled with a roll press to a positive electrode active material layer density of 1.9 g/cm$^3$, to obtain a positive electrode (P1) comprising a positive electrode active material layer and a positive electrode collector. The basis weight without the positive electrode collector was 17.5 mg/cm$^2$.

(3-1-2) Fabrication of positive electrode (P2)

(A) A complex oxide of lithium, nickel, manganese and cobalt with a number-mean particle size of 11 μm ($LiNi_{1/3}Mn_{1/3}Co_{1/3}O_2$, density: 4.70 g/cm$^3$), as a positive electrode active material, (B) graphite powder with a number-mean particle size of 6.5 μm (density: 2.26 g/cm$^3$) and acetylene black powder with a number-mean particle size of 48 nm (density: 1.95 g/cm$^3$), as conductive aids, and (C) polyvinylidene fluoride (PVDF, density: 1.75 g/cm$^3$) as a binder, were mixed in a weight ratio of (A) 92:(B) 4:(C) 4 to obtain a positive electrode mixture.

After then adding N-methyl-2-pyrrolidone as a solvent to the obtained positive electrode mixture to a solid content of 68 weight%, mixing was continued to prepare a positive electrode mixture-containing slurry. One side of an aluminum foil with a thickness of 15 μm and a width of 280 mm, serving as the positive electrode collector, was coated with the positive electrode mixture-containing slurry using a triple roll-type transfer coater, to a coating pattern with a coating width of 240 to 250 mm, a coating length of 125 mm and a non-coating length of 20 mm, while adjusting the basis weight, and the solvent was dried off with a hot air drying oven. The obtained electrode roll was trimmed on both sides and dried under reduced pressure at 130°C for 8 hours. It was then rolled with a roll press to a positive electrode active material layer density of 2.9 g/cm$^3$, to obtain a positive electrode (P2) comprising a positive electrode active material layer and a positive electrode collector. The basis weight of the positive electrode active material layer was 23.8 mg/cm$^2$.

(3-1-3) Fabrication of positive electrode (P3)

(A) A complex oxide of lithium, nickel, manganese and cobalt ($LiNi_{0.5}$Mn0.3 $Co_{0.2}O_2$), as a positive electrode active material, (B) acetylene black powder as a conductive aid and (C) polyvinylidene fluoride (PVDF) as a binder, were mixed in a weight ratio of 93.9:3.3:2.8 to obtain a positive electrode mixture.

After then adding N-methyl-2-pyrrolidone as a solvent to the obtained positive electrode mixture to a solid content of 68 weight%, mixing was continued to prepare a positive electrode mixture-containing slurry. One side of an aluminum foil with a thickness of 15 μm and a width of 280 mm, serving as the positive electrode collector, was coated with the positive electrode mixture-containing slurry using a triple roll-type transfer coater, to a coating pattern with a coating width of 240 to 250 mm, a coating length of 125 mm and a non-coating length of 20 mm, while adjusting the basis weight, and the solvent was dried off with a hot air drying oven. The obtained electrode roll was trimmed on both sides and dried under reduced pressure at 130°C for 8 hours. It was then rolled with a roll press to a positive electrode active material layer density of 2.7 g/cm$^3$, to obtain a positive electrode (P3) comprising a positive electrode active material layer and a positive electrode collector. The basis weight without the positive electrode collector was 9.3 mg/cm$^2$.

(3-1-4) Fabrication of positive electrode (P4)

(A) A complex oxide of lithium, nickel, manganese and cobalt ($LiNi_{0.8}Mn_{0.1}Co_{0.1}O_2$), as a positive electrode active material, (B) acetylene black powder as a conductive aid and (C) polyvinylidene fluoride (PVDF) as a binder, were mixed in a weight ratio of 94:3:3 to obtain a positive electrode mixture.

After then adding N-methyl-2-pyrrolidone as a solvent to the obtained positive electrode mixture to a solid

content of 68 weight%, mixing was continued to prepare a positive electrode mixture-containing slurry. One side of an aluminum foil with a thickness of 15 μm and a width of 280 mm, serving as the positive electrode collector, was coated with the positive electrode mixture-containing slurry using a triple roll-type transfer coater, to a coating pattern with a coating width of 240 to 250 mm, a coating length of 125 mm and a non-coating length of 20 mm, while adjusting the basis weight, and the solvent was dried off with a hot air drying oven. The obtained electrode roll was trimmed on both sides and dried under reduced pressure at 130°C for 8 hours. It was then rolled with a roll press to a positive electrode active material layer density of 2.9 g/cm$^3$, to obtain a positive electrode comprising a positive electrode active material layer and a positive electrode collector. The basis weight without the positive electrode collector was 16.6 mg/cm$^2$.

(3-2) Fabrication of negative electrode

(3-2-1) Fabrication of negative electrode (N1)

Graphite powder as a negative electrode active material, carbon black powder as a conductive aid, and carboxymethyl cellulose and styrene-butadiene rubber as binders, were mixed in a solid weight ratio of 95.7 (negative electrode active material):0.5 (conductive aid):3.8 (binder), to obtain a negative electrode mixture.

After then adding water as a solvent to the obtained negative electrode mixture to a solid content of 45 weight%, mixing was continued to prepare a negative electrode mixture-containing slurry. One side of a copper foil with a thickness of 8 μm and a width of 280 mm, serving as the negative electrode collector, was coated with the negative electrode mixture-containing slurry using a triple roll-type transfer coater, to a coating pattern with a coating width of 240 to 250 mm, a coating length of 125 mm and a non-coating length of 20 mm, while adjusting the basis weight, and the solvent was dried off with a hot air drying oven. The obtained electrode roll was trimmed on both sides and dried under reduced pressure at 80°C for 12 hours. It was then rolled with a roll press to a negative electrode active material layer density of 1.5 g/cm$^3$, to obtain a negative electrode (N1) comprising a negative electrode active material layer and a negative electrode collector. The basis weight without the negative electrode collector was 7.5 mg/cm$^2$.

(3-2-2) Fabrication of negative electrode (N2)

(a) Artificial graphite powder with a number-mean particle size of 12.7 μm (density: 2.23 g/cm$^3$), as a negative electrode active material, (b) acetylene black powder with a number-mean particle size 48 nm (density: 1.95 g/cm$^3$), as a conductive aid, and (c) carboxymethyl cellulose (density: 1.60 g/cm$^3$) solution (solid concentration: 1.83 weight%) and diene rubber (glass transition temperature: -5°C, dry number-mean particle size: 120 nm, density: 1.00 g/cm$^3$, dispersing medium: water, solid concentration: 40 weight%), as binders, were mixed in a solid weight ratio of (a) 95.7:(b) 0.5:(c) 3.8, to obtain a negative electrode mixture. After then adding water as a solvent to the obtained negative electrode mixture to a solid content of 45 weight%, mixing was continued to prepare a negative electrode mixture-containing slurry. One side of a copper foil with a thickness of 8 μm and a width of 280 mm, serving as the negative electrode collector, was coated with the negative electrode mixture-containing slurry using a triple roll-type transfer coater, to a coating pattern with a coating width of 240 to 250 mm, a coating length of 125 mm and a non-coating length of 20 mm, while adjusting the basis weight, and the solvent was dried off with a hot air drying oven. The obtained electrode roll was trimmed on both sides and dried under reduced pressure at 80°C for 12 hours. It was then rolled with a roll press to a negative electrode active material layer density of 1.5 g/cm$^3$, to obtain a negative electrode (N2) comprising a negative electrode active material layer and a negative electrode collector. The basis weight of the negative electrode active material layer was 11.9 mg/cm$^2$.

(3-2-3) Fabrication of negative electrode (N3)

(a) Graphite powder as a negative electrode active material and (c) a carboxymethyl cellulose (density: 1.60 g/cm$^3$) solution (solid concentration: 1.83 weight%) and diene rubber (glass transition temperature: -5°C, dry number-mean particle size: 120 nm, density: 1.00 g/cm$^3$, dispersing medium: water, solid concentration: 40 weight%), as binders, were mixed in a solid weight ratio of 97.4:1.1:1.5, to obtain a negative electrode mixture.

After then adding water as a solvent to the obtained negative electrode mixture to a solid content of 45 weight%, mixing was continued to prepare a negative electrode mixture-containing slurry. One side of a copper foil with a thickness of 8 μm and a width of 280 mm, serving as the negative electrode collector, was coated with the negative electrode mixture-containing slurry using a triple roll-type transfer coater, to a coating pattern with a coating width of 240 to 250 mm, a coating length of 125 mm and a non-coating length of 20 mm, while adjusting the basis weight, and the solvent was dried off with a hot air drying oven. The obtained electrode roll was trimmed on both sides and dried under reduced pressure at 80°C for 12 hours. It was then rolled with a roll press to a negative electrode active material layer density of 1.4 g/cm$^3$, to obtain a negative electrode (N3) comprising a negative electrode active material layer and a negative

electrode collector. The basis weight without the negative electrode collector was 5.9 mg/$cm^2$.

(3-2-4) Fabrication of negative electrode (N4)

(a) Graphite powder as a negative electrode active material, (b) acetylene black powder as a conductive aid and (c) polyvinylidene fluoride (PVDF) as a binder, were mixed in a weight ratio of 90.0:3.0:7.0 to obtain a negative electrode mixture.

After then adding water as a solvent to the obtained negative electrode mixture to a solid content of 45 weight%, mixing was continued to prepare a negative electrode mixture-containing slurry. One side of a copper foil with a thickness of 8 μm and a width of 280 mm, serving as the negative electrode collector, was coated with the negative electrode mixture-containing slurry using a triple roll-type transfer coater, to a coating pattern with a coating width of 240 to 250 mm, a coating length of 125 mm and a non-coating length of 20 mm, while adjusting the basis weight, and the solvent was dried off with a hot air drying oven. The obtained electrode roll was trimmed on both sides and dried under reduced pressure at 80°C for 12 hours. It was then rolled with a roll press to a negative electrode active material layer density of 1.4 g/$cm^3$, to obtain a negative electrode comprising a negative electrode active material layer and a negative electrode collector. The basis weight without the negative electrode collector was 10.3 mg/$cm^2$.

<First embodiment>

(3-3) Assembly of coin-type nonaqueous secondary battery

A polypropylene gasket was set in a CR2032 type battery case (SUS304/A1 cladding), and a positive electrode (P1) obtained as described above, punched into a discoid shape with a diameter of 15.958 mm, was set at the center with the positive electrode active material layer facing upward. Glass fiber filter paper (GA-100, product of Advantech, Inc.) punched out into a discoid shape with a diameter of 16.156 mm was then set over it, and 150 μL of a nonaqueous electrolyte solution (S1 to S3, S8, S23 to 25) was injected in, after which a negative electrode (N1) obtained as described above, punched into a discoid shape with a diameter of 16.156 mm, was set with the negative electrode active material layer facing downward. After further setting a spacer and spring inside the battery case, a battery cap was fitted over and a caulking seal was formed with a caulking machine. The overflowing electrolyte solution was wiped off with a waste cloth. The battery was stored at 25°C for 12 hours to allow sufficient interaction of the nonaqueous electrolyte solution with the layered product, to obtain a coin-type nonaqueous secondary battery (P1/N1).

The same procedure was used to obtain a coin-type nonaqueous secondary battery using P2 for the positive electrode, N2 for the negative electrode, and S4 to S6 and S23 to S26 for the electrolyte solution (P2/N2), a coin-type nonaqueous secondary battery using P3 for the positive electrode, N3 for the negative electrode, and S4, S7, S32 and S36 to S40 for the electrolyte solution (P3/N3), and a coin-type nonaqueous secondary battery using P4 for the positive electrode, N4 for the negative electrode, and S8, S9, S24 and S41 for the electrolyte solution (P4/N4).

(4) Evaluation of coin-type nonaqueous secondary batteries

For the coin-type nonaqueous secondary batteries (P1/N1) obtained above (Examples 1 to 3 and Comparative Examples 1 to 3), first initial charge processing and initial charge-discharge capacity measurement were carried out by the procedure in (4-1) below. The coin-type nonaqueous secondary batteries were then evaluated by the procedures in (4-2) and (4-3). Charge-discharge was carried out using an ACD-M01A charge-discharge device (trade name of Aska Electronic Co., Ltd.), and an IN804 programmable thermostatic bath (trade name of Yamato Scientific Co., Ltd.).

As used herein, "1 C" means the current value expected when a battery at full charge is discharged at constant current for 1 hour to complete discharge.

Specifically, for the coin-type nonaqueous secondary battery (P1/N1), "1 C" means the current value expected after discharge from a full charge of 3.8 V to 2.5 V at constant current for 1 hour to complete discharge.

For the coin-type nonaqueous secondary battery (P2/N2), the coin-type nonaqueous secondary battery (P3/N3) and the coin-type nonaqueous secondary battery (P4/N4), "1 C" means the current value expected after discharge from a full charge of 4.2 V to 3.0 V at constant current for 1 hour to complete discharge.

The coin-type nonaqueous secondary battery (P1/N1) assembled by the procedure in (3-3) above was a 4.6 mAh-class cell, the full charge cell voltage was set to 3.8 V, and the current value corresponding to 1 C was 4.6 mA. The coin-type nonaqueous secondary battery (P2/N2) and coin-type nonaqueous secondary battery (P4/N4) were 6 mAh class cells, the full charge cell voltage was set to 4.2 V, and the current value corresponding to 1 C was 6.0 mA. The coin-type nonaqueous secondary battery (P3/N3) was a 3 mAh-class cell, the full charge cell voltage was set to 4.2 V, and the current value corresponding to 1 C was 3.0 mA. For convenience, the current values and voltages will not be specified below unless necessary.

(4-1) Initial charge-discharge processing for coin-type nonaqueous secondary battery

The ambient temperature for the coin-type nonaqueous secondary battery (P1/N1) was set to 25°C, and after charging at a constant current corresponding to 0.1 C and reaching a state of full charge, charging was carried out for 1.5 hours at constant voltage. The battery was then discharged to a prescribed voltage at a constant current corresponding to 0.3 C. The discharge capacity during this time was divided by the charge capacity to calculate the initial efficiency. The discharge capacity at that time was recorded as the initial capacity. Initial charge-discharge processing was carried out in the same manner for the coin-type nonaqueous secondary battery (P2/N2), coin-type nonaqueous secondary battery (P3/N3) and coin-type nonaqueous secondary battery (P4/N4).

(4-2) Full charge storage test at 85°C for coin-type nonaqueous secondary battery (4-2-1) Full charge storage test at 85°C for coin-type nonaqueous secondary battery (P1/N1)

The coin-type nonaqueous secondary battery (P1/N1) subjected to initial charge-discharge processing by the method described in (4-1) above was charged at a constant current of 4.6 mA corresponding to 1 C with the ambient temperature set to 25°C, and after reaching 3.8 V, it was charged for 1.5 hours at a constant voltage of 3.8 V. The coin-type nonaqueous secondary battery was then stored for 4 hours in a thermostatic bath at 85°C. The ambient temperature was returned to 25°C, and discharge was carried out to 2.5 V at a current value of 1.38 mA corresponding to 0.3 C. The residual discharge capacity at that time was recorded as the 0.3 C residual capacity. The coin-type nonaqueous secondary battery (P1/N1) that had been subjected to full charge storage test at 85°C by the method described above was charged at a constant current of 4.6 mA corresponding to 1 C with the ambient temperature set to 25°C, and after reaching 3.8 V, it was charged for 1.5 hours at a constant voltage of 3.8 V. The charge capacity during this time was recorded as the 1 C recovery charge capacity. Discharge was then carried out to 2.5 V at a current value of 1.38 mA corresponding to 0.3 C. The discharge capacity during this time was recorded as the 0.3 C recovery discharge capacity. After then further charging at a constant current of 4.6 mA corresponding to 1 C and reaching 3.8 V, charge was carried out for 1.5 hours at a constant voltage of 3.8 V. Discharge was then carried out to 2.5 V at a current value of 6.9 mA corresponding to 1.5 C. The discharge capacity during this time was recorded as the 1.5 C recovery discharge capacity.

(4-2-2) Full charge storage test at 85°C for coin-type nonaqueous secondary battery (P2/N2)

The coin-type nonaqueous secondary battery (P2/N2) subjected to initial charge-discharge processing by the method described in (4-1) above was charged at a constant current of 6.0 mA corresponding to 1 C with the ambient temperature set to 25°C, and after reaching 4.2 V, it was charged for 1.5 hours at a constant voltage of 4.2 V. The coin-type nonaqueous secondary battery was then stored for 4 hours in a thermostatic bath at 85°C. The ambient temperature was returned to 25°C, and discharge was carried out to 3.0 V at a current value of 1.8 mA corresponding to 0.3 C. The residual discharge capacity at that time was recorded as the 0.3 C residual capacity. The coin-type nonaqueous secondary battery (P2/N2) that had been subjected to full charge storage test at 85°C by the method described above was charged at a constant current of 6.0 mA corresponding to 1 C with the ambient temperature set to 25°C, and after reaching 4.2 V, it was charged for 1.5 hours at a constant voltage of 4.2 V. The charge capacity during this time was recorded as the 1 C recovery charge capacity. Discharge was then carried out to 3.0 V at a current value of 1.8 mA corresponding to 0.3 C. The discharge capacity during this time was recorded as the 0.3 C recovery discharge capacity. After then further charging at a constant current of 6.0 mA corresponding to 1 C and reaching 4.2 V, charge was carried out for 1.5 hours at a constant voltage of 4.2 V. Discharge was then carried out to 3.0 V at a current value of 9.0 mA corresponding to 1.5 C. The discharge capacity during this time was recorded as the 1.5 C recovery discharge capacity.

(4-2-3) Full charge storage test at 85°C for coin-type nonaqueous secondary battery (P3/N3)

The coin-type nonaqueous secondary battery (P3/N3) subjected to initial charge-discharge processing by the method described in (4-1) above was charged at a constant current of 3.0 mA corresponding to 1 C with the ambient temperature set to 25°C, and after reaching 4.2 V, it was charged for 1.5 hours at a constant voltage of 4.2 V. The coin-type nonaqueous secondary battery was then stored for 4 hours in a thermostatic bath at 85°C. The ambient temperature was returned to 25°C, and discharge was carried out to 3.0 V at a current value of 0.9 mA corresponding to 0.3 C. The residual discharge capacity at that time was recorded as the 0.3 C residual capacity. The coin-type nonaqueous secondary battery (P3/N3) that had been subjected to full charge storage test at 85°C by the method described above was charged at a constant current of 3.0 mA corresponding to 1 C with the ambient temperature set to 25°C, and after reaching 4.2 V, it was charged for 1.5 hours at a constant voltage of 4.2 V. The charge capacity during this time was recorded as the 1 C recovery charge capacity. Discharge was then carried out to 3.0 V at a current value of 0.9 mA corresponding to 0.3 C. The discharge capacity during this time was recorded as the 0.3 C recovery discharge capacity. After then further

charging at a constant current of 3.0 mA corresponding to 1 C and reaching 4.2 V, charge was carried out for 1.5 hours at a constant voltage of 4.2 V. Discharge was then carried out to 3.0 V at a current value of 9 mA corresponding to 3 C. The discharge capacity during this time was recorded as the 3 C recovery discharge capacity.

(4-3) Calculation of measurement values for full charge storage test at 85°C

The 0.3 C residual capacity retention rate, the post-recovery charge-discharge efficiency and the recovery capacity retention rate, for the coin-type nonaqueous secondary batteries that had been subjected to a full charge storage test at 85°C by the method described in (4-2-1) to (4-2-3), were calculated by the following formulas, as the measurement values for the full charge storage test at 85°C. The results are shown in Tables 2 to 4.

$$\text{0.3 C Residual capacity retention rate} = (\text{0.3 C Residual discharge capacity after 85°C full charge and storage/initial capacity before 85°C full charge storage test}) \times 100\ [\%]$$

$$\text{Post-recovery charge-discharge efficiency} = (\text{0.3 C Recovery discharge capacity after 85°C full charge storage test/1 C recovery charge capacity after 85°C full charge storage test}) \times 100\ [\%]$$

$$\text{0.3 C Recovery capacity retention rate} = (\text{0.3 C Recovery discharge capacity after 85°C full charge storage test/initial capacity before 85°C full charge storage test}) \times 100\ [\%]$$

$$\text{1.5 C Recovery capacity retention rate} = (\text{1.5 C Recovery discharge capacity after 85°C full charge storage test/initial capacity before 85°C full charge storage test}) \times 100\ [\%]$$

(4-3-1) Coin-type nonaqueous secondary battery (P1/N1)

[Examples 1 to 3 and Comparative Examples 1 to 3]

The test results may be interpreted as follows.

The first initial charge-discharge efficiency represents the ratio of the initial discharge capacity with respect to the initial charge capacity, and it is generally lower than the second charge-discharge efficiency onward. This is due to formation of negative electrode SEI with Li ions during the initial charge. The number of Li ions that can be discharged is therefore reduced. The first initial charge-discharge efficiency is not problematic if it is 84% or higher.

The 0.3 C residual capacity retention rate is an index of the amount of self-discharge during the full charge storage test at 85°C. A larger value corresponds to lower self-discharge at high temperature, allowing more current to be used. Since an iron-based positive electrode with an olivine structure is more stable in high-temperature environments than a complex oxide-based positive electrode comprising lithium, nickel, manganese and cobalt, the 0.3 C residual capacity retention rate is preferably 88% or higher, more preferably 90% or higher and even more preferably 92% or higher.

The charge-discharge efficiency is preferably 95% or higher, more preferably 98% or higher and even more preferably 99% or higher, so that it is at least equivalent to the charge-discharge efficiency of a common nonaqueous secondary battery.

The 0.3 C recovery capacity retention rate is an index of the output during use with low electrification current. With 0.3 C, the capacity retention is preferably 90% or higher and more preferably 92% or higher, since it will be less likely to be affected by internal resistance in the battery.

The 1.5 C recovery capacity retention rate is an index of the output during use with high electrification current. With 1.5 C, the recovery capacity retention rate is more likely to be affected by the internal resistance of the battery, and is therefore lower than 0.3 C. Therefore, the 1.5 C recovery capacity retention rate is preferably 88% or higher, more preferably 90% or higher and even more preferably 92% or higher.

Table 2

| | Electrolyte solution No. | Nitrogen-containing heterocyclic ring compound | | First initial charge-discharge efficiency [%] | 0.3 C Residual capacity retention rate [%] | Charge-discharge efficiency [%] | 0.3 C Recovery capacity retention rate [%] | 1.5 C Recovery capacity retention rate [%] |
|---|---|---|---|---|---|---|---|---|
| | | Type | Addition amount [parts by weight] | | | | | |
| Example 1 | S1 | CAF | 0.25 | 91.6 | 92.9 | 99.6 | 94.1 | 93.1 |
| Example 2 | S2 | CAF | 0.25 | 91.8 | 93.0 | 99.8 | 93.9 | 93.2 |
| Example 3 | S3 | CAF | 0.25 | 90.8 | 94.0 | 99.9 | 94.8 | 93.0 |
| Comparative Example 1 | S23 | CAF | 0.25 | 91.9 | 91.5 | 99.7 | 93.8 | 91.1 |
| Comparative Example 2 | S24 | - | - | 91.5 | 91.4 | 99.7 | 94.2 | 90.7 |
| Comparative Example 3 | S25 | - | - | 92.1 | 86.3 | 99.4 | 92.2 | 89.4 |

The results were all on an acceptable level in all of the tests in Examples 1 to 3. In Comparative Examples 2 to 3, on the other hand, which were common carbonate electrolyte solutions, the residual capacity retention rate and 1.5 C recovery rate were significantly inferior compared to Examples 1 to 3. In Comparative Example 1, the results did not reach the high temperature storage property of the embodiment even though a condensation polycyclic heterocyclic ring compound similar to that of the electrolyte solution of the embodiment was added to the nonaqueous electrolyte solution used in Comparative Example 2. Since a condensation polycyclic compound is expected to have an effect of inhibiting generation of complex cations comprising transition metals and acetonitrile, its combination with a nonaqueous electrolyte solution that contains acetonitrile as with this embodiment may improve performance more effectively.

(4-3-2) Coin-type nonaqueous secondary battery (P2/N2)

[Examples 4 to 6 and Comparative Examples 4 to 17]

For a coin-type nonaqueous secondary battery (P2/N2), there is no particular problem if the first initial charge-discharge efficiency is 84% or higher. Therefore, the 0.3 C residual capacity retention rate is preferably 80% or higher, more preferably 85% or higher and even more preferably 90% or higher. The charge-discharge efficiency is preferably 95% or higher, more preferably 98% or higher and even more preferably 99% or higher. The 0.3 C recovery capacity retention rate is preferably 90% or higher and more preferably 92% or higher. The 1.5 C recovery capacity retention rate is preferably 88% or higher, more preferably 89% or higher and even more preferably 90% or higher.

[Table 3]

| | Electrolyte solution No. | Nitrogen-containing heterocyclic ring compound | | First initial charge-discharge efficiency [%] | 0.3 C Residual capacity retention rate [%] | Charge-discharge efficiency [%] | 0.3 C Recovery capacity retention rate [%] | 1.5 C Recovery capacity retention rate [%] |
|---|---|---|---|---|---|---|---|---|
| | | Type | Addition amount [parts by weight] | | | | | |
| Example 4 | S4 | CAF | 0.25 | 86.2 | 92.2 | 99.5 | 98.5 | 91.9 |
| Example 5 | S5 | CAF | 0.12 | 87.4 | 93.3 | 98.0 | 100.6 | 91.1 |
| Example 6 | S6 | CAF | 0.10 | 88.7 | 91.8 | 99.1 | 100.7 | 93.7 |
| Comparative Example 4 | S26 | PZP | 0.10 | 85.0 | 57.6 | 74.6 | 56.1 | 39.1 |
| Comparative Example 5 | S27 | DPP | 0.10 | 87.4 | 83.0 | 85.7 | 80.2 | 66.4 |
| Comparative Example 6 | S28 | DPPZ | 0.10 | 86.6 | 89.5 | 92.1 | 89.3 | 77.3 |
| Comparative Example 7 | S29 | PPZ | 0.10 | 86.1 | 75.9 | 81.0 | 70.6 | 54.1 |
| Comparative Example 8 | S30 | EPZ | 0.10 | 87.4 | 89.5 | 92.1 | 90.1 | 78.9 |
| Comparative Example 9 | S31 | INZ | 0.10 | 86.1 | 87.7 | 92.1 | 88.6 | 76.1 |
| Comparative Example 10 | S32 | MBTA | 0.25 | 86.2 | 88.4 | 97.8 | 97.7 | 89.4 |
| Comparative Example 11 | S33 | MBTA | 0.50 | 88.3 | 89.4 | 99.3 | 99.8 | 92.8 |
| Comparative Example 12 | S34 | PD | 0.10 | 87.1 | 88.8 | 99.4 | 97.8 | 90.6 |
| Comparative Example 13 | S35 | PD | 0.25 | 87.8 | 86.9 | 99.4 | 97.8 | 91.7 |
| Comparative Example 14 | S36 | - | - | 87.6 | 90.7 | 92.0 | 90.4 | 78.3 |

(continued)

| | Electrolyte solution No. | Nitrogen-containing heterocyclic ring compound | | First initial charge-discharge efficiency [%] | 0.3 C Residual capacity retention rate [%] | Charge-discharge efficiency [%] | 0.3 C Recovery capacity retention rate [%] | 1.5 C Recovery capacity retention rate [%] |
|---|---|---|---|---|---|---|---|---|
| | | Type | Addition amount [parts by weight] | | | | | |
| Comparative Example 15 | S23 | CAF | 0.25 | 88.5 | 86.5 | 99.8 | 100.6 | 74.1 |
| Comparative Example 16 | S24 | - | - | 88.5 | 86.2 | 99.4 | 98.3 | 67.9 |
| Comparative Example 17 | S25 | - | - | 86.2 | 86.6 | 99.9 | 99.6 | 75.2 |

Examples 4 to 6 used CAF, and satisfied all of the evaluation criteria. In Comparative Examples 4 to 9, however, which contained other nitrogen-containing heterocyclic ring compounds, the self-discharge during storage testing was high, and the residual capacity retention rate was lowered. In Comparative Examples 10 to 13, the 1.5 C recovery capacity retention rate was high and the additives produced an inhibiting effect on increased internal resistance, but the residual capacity retention rate was low and inhibition of self-discharge was inadequate. Comparative Example 14 had a high residual capacity retention rate, but the 1.5 C recovery capacity retention rate was significantly lowered and there was concern regarding insufficient output due to increased internal resistance during storage. Comparative Examples 15 to 17 did not contain acetonitrile, and the Li ion diffusion in the electrolyte solution was slow. Therefore, the 1.5 C recovery capacity retention rate was significantly lowered when using a thick-film positive electrode as with P2.

(4-3-3) Coin-type nonaqueous secondary battery (P3/N3)

[Examples 7 and 8, and Comparative Examples 18 to 23]

For a coin-type nonaqueous secondary battery (P3/N3), there is no particular problem if the first initial charge-discharge efficiency is 80% or higher. The 0.3 C residual capacity retention rate is preferably 75% or higher, more preferably 78% or higher and even more preferably 80% or higher. The charge-discharge efficiency is preferably 95% or higher, more preferably 98% or higher and even more preferably 99% or higher. The 0.3 C recovery capacity retention rate is preferably 90% or higher, more preferably 95% or higher and even more preferably 98% or higher. The 1.5 C recovery capacity retention rate is preferably 80% or higher, more preferably 82% or higher and even more preferably 84% or higher.

[Table 4]

| | Electrolyte solution No. | Nitrogen-containing heterocyclic ring compound | | First initial charge-discharge efficiency [%] | 0.3 C Residual capacity retention rate [%] | Charge-discharge efficiency [%] | 0.3 C Recovery capacity retention rate [%] | 3 C Recovery capacity retention rate [%] |
|---|---|---|---|---|---|---|---|---|
| | | Type | Addition amount [parts by weight] | | | | | |
| Example 7 | S7 | CAF | 0.10 | 84.3 | 80.0 | 99.6 | 98.8 | 84.0 |
| Example 8 | S4 | CAF | 0.25 | 84.5 | 80.7 | 99.4 | 98.9 | 84.0 |
| Comparative Example 18 | S36 | - | - | 84.4 | Abnormal capacity | - | - | - |
| Comparative Example 19 | S37 | MPPZ | 0.10 | 82.4 | 76.2 | 100.2 | 98.1 | 82.7 |
| Comparative Example 20 | S38 | MPPZ | 0.25 | 80.0 | 79.4 | 100.4 | 97.0 | 80.9 |
| Comparative Example 21 | S39 | MPPZ | 0.50 | 73.6 | 68.1 | 100.5 | 99.3 | 79.0 |
| Comparative Example 22 | S40 | MBTA | 0.10 | 84.4 | 78.5 | 99.6 | 99.5 | 84.8 |
| Comparative Example 23 | S32 | MBTA | 0.25 | 82.2 | 79.9 | 99.5 | 98.6 | 74.7 |

Examples 7 and 8 satisfied all of the criteria for the embodiment. However, Comparative Examples 19 to 23 which used electrolyte solutions containing nitrogen-containing heterocyclic ring compounds other than a nitrogen-containing heterocyclic ring compound specified by the embodiment, had high self-discharge during storage testing, and lowered residual capacity retention rates. The coin-type nonaqueous secondary battery of Comparative Example 18 exhibited abnormal capacity and ceased to function, in the charge-discharge evaluation after storage.

(4-4) Discharge test at -40°C

The coin-type nonaqueous secondary battery (P4/N4) subjected to initial charge-discharge processing by the method described in (4-1) above was charged at a constant current of 2.3 mA corresponding to 0.5 C with the ambient temperature of the battery set to 25°C, and after reaching 3.8 V, it was charged at a constant voltage of 3.8 V until the current was attenuated to 0.05 C, and then discharged to 2.5 V at a current value of 0.46 mA corresponding to 0.1 C. After then further charging at a constant current of 2.3 mA corresponding to 0.5 C and reaching 3.8 V, charge was carried out at a constant voltage of 3.8 V, until the current was attenuated to 0.05 C. The ambient temperature of the battery was then set to -40°C, and after a standby time of 3 hours, discharge was carried out to 2.5 V at a current value of 0.46 mA corresponding to 0.1 C.

The discharge capacity at -40°C, assuming a discharge capacity of 100% in an environment of 25°C, was calculated as the capacity retention. The results are shown in Table 5.

[Table 5]

| | Electrolyte solution No. | -40°C Discharge capacity retention rate (%) |
|---|---|---|
| Example 9 | S8 | 58.7 |
| Comparative Example 24 | S24 | 12.8 |

As shown in Table 5, Example 9 which used a nonaqueous electrolyte solution containing acetonitrile as the nonaqueous solvent exhibited a capacity retention of ≥50% in the discharge test at -40°C, which was an excellent low-temperature characteristic. In Comparative Example 24 which used a carbonate electrolyte solution, however, the capacity retention in the discharge test at -40°C was significantly lowered, and low temperature performance was not satisfactorily exhibited. The nonaqueous electrolyte solution of the embodiment was confirmed to exhibit both high temperature durability and low temperature operability.

(4-5) Cycle test at 50°C for coin-type nonaqueous secondary battery

(4-5-1) Cycle test at 50°C for coin-type nonaqueous secondary battery (P1/N1)

For the coin-type nonaqueous secondary battery (P1/N1) which had been subjected to initial charge-discharge processing by the method described in (4-1) above, the ambient temperature was set to 50°C. First, the battery was charged at a constant current of 6.9 mA corresponding to 1.5 C, and after reaching 3.8 V, charge was carried out at a constant voltage of 3.8 V, until the current was attenuated to 0.23 mA corresponding to 0.05 C. The battery was then discharged to 2.5 V at a constant current of 6.9 mA. With the step of a single charge and discharge defined as one cycle, charge-discharge was carried out for 100 cycles. The discharge capacity at the 100th cycle was recorded as the capacity retention, with the discharge capacity in the first cycle as 100%. The results are shown in Table 6.

[Table 6]

| | Electrolyte solution No. | Initial efficiency (%) | Capacity retention rate (%) |
|---|---|---|---|
| Example 10 | S1 | 91.3 | 92.4 |
| Example 11 | S2 | 91.5 | 91.7 |
| Comparative Example 25 | S24 | 90.3 | 88.4 |
| Comparative Example 26 | S25 | 91.6 | 86.7 |

As shown in Table 6, the capacity reduction was low after cycling at high temperature in Examples 10 to 11, thus confirming improved cycle performance.

(4-5-2) Prolonged cycle test at 50°C for coin-type nonaqueous secondary battery (P1/N1)

The coin-type nonaqueous secondary batteries (P1/N1) of Example 10 and Comparative Example 25, which were subjected to 100 cycles in (4-5-1) above, were further subjected to 300 continuous cycles, and were used as secondary batteries for Example 12 and Comparative Example 27, respectively. The discharge capacity at the 400th cycle was recorded as the capacity retention, with the discharge capacity in the first cycle as 100%. The results are shown in Table 7.

[Table 7]

| | Electrolyte solution No. | Initial efficiency (%) | Capacity retention rate (%) |
|---|---|---|---|
| Example 12 | S1 | 91.3 | 82.0 |
| Comparative Example 27 | S24 | 91.6 | 78.8 |

As shown in Table 7, comparison between Example 12 and Comparative Example 27 allowed confirmation that the coin-type nonaqueous secondary battery (P1/N1) of Example 12 had an improved lifetime characteristic in the prolonged usable life evaluation test.

(4-5-3) Cycle test at 50°C for coin-type nonaqueous secondary battery (P4/N4)

For the coin-type nonaqueous secondary battery (P4/N4) which had been subjected to initial charge-discharge processing by the method described in (4-1) above, the ambient temperature was set to 50°C. After first charging at a constant current of 9.0 mA corresponding to 1.5 C and reaching 4.2 V, charge was carried out at a constant voltage of 4.2 V, until the current was attenuated to 0.30 mA corresponding to 0.05 C. The battery was then discharged to 3.0 V at a constant current of 9.0 mA. With the step of a single charge and discharge defined as one cycle, charge-discharge was carried out for 100 cycles. The discharge capacity at the 100th cycle was recorded as the capacity retention, with the discharge capacity in the first cycle as 100%. The results are shown in Table 8.

[Table 8]

| | Electrolyte solution No. | Initial efficiency (%) | Residual capacity retention rate (%) |
|---|---|---|---|
| Example 13 | S9 | 85.0 | 88.7 |
| Comparative Example 28 | S41 | 85.5 | 83.3 |
| Comparative Example 29 | S24 | 85.9 | 87.4 |

As shown in Table 8, the coin-type nonaqueous secondary battery (P4/N4) of Example 13 has low reduction in capacity after the cycle test at high temperature, thus confirming improved cycle performance.

<Second embodiment>

(5-1) Assembly of coin-type nonaqueous secondary battery (P1/N1)

A polypropylene gasket was set in a CR2032 type battery case (SUS304/A1 cladding), and a positive electrode (P1) obtained as described above, punched into a discoid shape with a diameter of 15.958 mm, was set at the center with the positive electrode active material layer facing upward. Glass fiber filter paper (GA-100, product of Advantech, Inc.) punched out into a discoid shape with a diameter of 16.156 mm was then set over it, and 150 μL of a nonaqueous electrolyte solution (S10 to S13, S23 to 25, S42, S43) was injected in, after which a negative electrode (N1) obtained as described above, punched into a discoid shape with a diameter of 16.156 mm, was set with the negative electrode active material layer facing downward. After further setting a spacer and spring inside the battery case, a battery cap was fitted over and a caulking seal was formed with a caulking machine. The overflowing electrolyte solution was wiped off with a waste cloth. The battery was stored at 25°C for 12 hours to allow sufficient interaction of the nonaqueous electrolyte solution with the layered product, to obtain a coin-type nonaqueous secondary battery (P1/N1).

(5-2) Initial charge-discharge processing for coin-type nonaqueous secondary battery (P1/N1)

The coin-type nonaqueous secondary battery (P1/N1) obtained in (5-1) was subjected to initial charge-discharge

processing by the same procedure as (4-1), and the initial efficiency was calculated.

(5-3) Full charge storage test at 85°C for coin-type nonaqueous secondary battery (P1/N1)

The coin-type nonaqueous secondary battery (P1/N1) that had been subjected to initial charge-discharge processing by the method described in (5-2) above was subjected to a full charge storage test at 85°C by the method described in (4-2-1) above, and this was followed by battery performance calculation by the methods described in (4-3) above. The results are shown in Table 9. Interpretation of the test results for Examples 14 to 16 is as explained in (4-3-1) above.

[Table 9]

| | Electrolyte solution No. | Nitrogen-containing heterocyclic ring compound | | First initial charge-discharge efficiency [%] | 0.3 C Residual capacity retention rate [%] | Charge-discharge efficiency [%] | 0.3 C Recovery capacity retention rate [%] | 1.5 C Recovery capacity retention rate [%] |
|---|---|---|---|---|---|---|---|---|
| | | Type | Addition amount [parts by weight] | | | | | |
| Example 14 | S10 | CAF | 0.25 | 91.9 | 95.1 | 100.0 | 95.9 | 95.2 |
| Example 15 | S11 | CAF | 0.25 | 90.5 | 94.3 | 99.9 | 95.3 | 94.4 |
| Example 16 | S12 | CAF | 0.25 | 91.7 | 94.2 | 99.9 | 95.4 | 94.6 |

(5-4) Discharge test at -40°C for coin-type nonaqueous secondary battery (P1/N1)

The coin-type nonaqueous secondary battery (P1/N1) that had been subjected to initial charge-discharge processing by the method described in (5-2) above was subjected to a discharge test at -40°C by the method described in (4-4) above, and the discharge capacity at -40°C, assuming 100% discharge capacity and an environment of 25°C, was calculated as the capacity retention. The results are shown in Table 10.

Table 10

| | Electrolyte solution No. | -40°C Discharge capacity retention rate (%) |
|---|---|---|
| Example 17 | S12 | 59.7 |
| Example 18 | S13 | 59.5 |

As shown in Table 10, Examples 17 and 18 which used a nonaqueous electrolyte solution containing acetonitrile as the nonaqueous solvent exhibited a capacity retention of ≥50% in the discharge test at -40°C, which was an excellent low-temperature characteristic. Thus, both high temperature durability and low temperature operability were confirmed for this embodiment as well.

(5-5) Cycle test at 50°C for coin-type nonaqueous secondary battery (P1/N1)

(5-5-1) Cycle test at 50°C for coin-type nonaqueous secondary battery (P1/N1)

The coin-type nonaqueous secondary battery (P1/N1) which had been subjected to initial charge-discharge processing by the method described in (5-2) above was subjected to a cycle test at 50°C by the method described in (4-5-1) above. The results are shown in Table 11.

[Table 11]

| | Electrolyte solution No. | Initial efficiency (%) | Capacity retention rate (%) |
|---|---|---|---|
| Example 19 | S12 | 91.4 | 92.0 |
| Example 20 | S13 | 91.7 | 92.4 |
| Comparative Example 30 | S24 | 90.3 | 88.4 |
| Comparative Example 31 | S25 | 91.6 | 86.7 |

(5-5-2) Prolonged cycle test at 50°C for coin-type nonaqueous secondary battery (P1/N1)

The coin-type nonaqueous secondary batteries (P1/N1) of Example 19 and Comparative Example 30, which were subjected to 100 cycles in (5-5-1) above, were further subjected to 300 continuous cycles, and were used as secondary batteries for Example 21 and Comparative Example 32, respectively. The discharge capacity at the 400th cycle was recorded as the capacity retention, with the discharge capacity in the first cycle as 100%. The results are shown in Table 12.

[Table 12]

| | Electrolyte solution No. | Initial efficiency (%) | Capacity retention rate (%) |
|---|---|---|---|
| Example 21 | S12 | 91.4 | 83.2 |
| Comparative Example 32 | S24 | 90.3 | 78.8 |

As shown in Table 12, the coin-type nonaqueous secondary battery of Example 21 had no significant reduction in capacity retention even in the prolonged usable life evaluation test, and thus maintained a high lifetime characteristic.

(6-1) 10-day and 30-day full charge storage tests at 85°C for coin-type nonaqueous secondary battery (P1/N1)

The coin-type nonaqueous secondary battery (P1/N1) subjected to initial charge-discharge processing by the method described in (5-2) above was charged at a constant current corresponding to 1 C with the ambient temperature set to 25°C, and after reaching a full charge state, it was charged for 1.5 hours at a constant voltage. The coin-type nonaqueous secondary battery was then stored for 10 days in a thermostatic bath at 85°C. The ambient temperature was returned to 25°C, and the battery was discharged to a prescribed voltage at a current value corresponding to 0.1 C. The residual discharge capacity at that time was recorded as the 10-day residual discharge capacity. After then further charging at a constant current corresponding to 0.1 C and reaching a full charge state, charge was carried out for 1.5 hours at a constant voltage. The recovery charge capacity during this time was recorded as the 10-day recovery charge capacity. Discharge was then carried out to a prescribed voltage at a current value corresponding to 0.1 C. The recovery discharge capacity during this time was recorded as the 10-day recovery discharge capacity. The residual capacity retention rate, charge-discharge efficiency and recovery capacity retention rate were calculated by the following formulas, as measurement values in the 10-day full charge storage test at 85°C.

$$\text{10-Day residual capacity retention rate} = (\text{10-day residual discharge capacity}/\text{initial capacity}) \times 100\ [\%]$$

$$\text{10-Day post-recovery efficiency} = (\text{10-day recovery discharge capacity}/\text{10-day recovery charge capacity}) \times 100\ [\%]$$

$$\text{10-Day recovery capacity retention rate} = (\text{10-day recovery discharge capacity}/\text{initial capacity}) \times 100\ [\%]$$

The coin-type nonaqueous secondary battery was then charged at a constant current corresponding to 1 C, and after reaching a full charge state, it was charged for 1.5 hours at a constant voltage. The coin-type nonaqueous

secondary battery was then stored for 20 days in a thermostatic bath at 85°C. The ambient temperature was returned to 25°C, and discharge was carried out to a prescribed voltage at a current value corresponding to 0.1 C. The residual discharge capacity at that time was recorded as the 30-day residual discharge capacity. After then further charging at a constant current corresponding to 0.1 C and reaching a full charge state, charge was carried out for 1.5 hours at a constant voltage. The recovery charge capacity during this time was recorded as the 30-day recovery charge capacity. Discharge was then carried out to a prescribed voltage at a current value corresponding to 0.1 C. The recovery discharge capacity during this time was recorded as the 25-day recovery discharge capacity. The residual capacity retention rate, charge-discharge efficiency and recovery capacity retention rate were calculated by the following formulas, as measurement values in the 30-day full charge storage test at 85°C.

$$\text{30-Day residual capacity retention rate} = (\text{30-day residual discharge capacity}/\text{initial capacity}) \times 100\ [\%]$$

$$\text{30-Day post-recovery efficiency} = (\text{30-day recovery discharge capacity}/\text{30-day recovery charge capacity}) \times 100\ [\%]$$

$$\text{30-Day recovery capacity retention rate} = (\text{30-day recovery discharge capacity}/\text{initial capacity}) \times 100\ [\%]$$

The test results may be interpreted as follows.

First, the residual capacity retention rate is an index of the size of self-discharge in the full charge storage test at 85°C. A larger value corresponds to lower self-discharge at high temperature, allowing more current to be used. The 10-day residual capacity retention rate is preferably 30% or higher and more preferably 40% or higher. The 30-day residual capacity retention rate is preferably 5% or higher and more preferably 10% or higher.

The post-recovery efficiency is an index of continuous degradation in the cell after the full charge storage test at 85°C. The post-recovery efficiency is preferably 98.0% or higher both after 10 days and after 30 days, and if it satisfies this condition then it can be used subsequently in a 30-day full charge storage test. The post-recovery efficiency is more preferably 99.0% or higher and even more preferably 99.5% or higher.

The recovery capacity retention rate is an index of the irreversible capacity in the storage test. A larger value means a lower amount of lithium that is irreversibly consumed in the storage test, allowing more current to be used even after prolonged exposure in a high-temperature environment. The 10-day recovery capacity retention rate is preferably 40% or higher, more preferably 50% or higher and even more preferably 60% or higher. The 30-day recovery capacity retention rate is preferably 15% or higher, more preferably 20% or higher and even more preferably 25% or higher.

Table 13 shows the results of the 10-day and 30-day full charge storage tests.

[Table 13]

| | Electrolyte solution No. | Initial efficiency (%) | After 10 days | | | After 30 days | | |
|---|---|---|---|---|---|---|---|---|
| | | | Residual capacity retention rate (%) | Post-recovery efficiency (%) | Recovery capacity retention rate (%) | Residual capacity retention rate (%) | Post-recovery efficiency (%) | Recovery capacity retention rate (%) |
| Example 22 | S1 | 90.8 | 47.0 | 100.2 | 60.1 | 12.7 | 99.6 | 27.3 |
| Comparative Example 33 | S42 | 88.9 | 28.6 | 98.1 | 39.8 | Abnormal voltage | - | - |
| Comparative Example 34 | S43 | 90.1 | 48.1 | 97.6 | 62.4 | - | - | - |
| Comparative Example 35 | S23 | 88.8 | 51.3 | 97.2 | 61.8 | - | - | - |
| Comparative Example 36 | S24 | 88.9 | 56.5 | 98.4 | 66.2 | 1.3 | 97.3 | 21.4 |

As shown in Table 13, with Example 22 the residual capacity retention rate, recovery capacity retention rate and post-recovery efficiency satisfied the acceptability level after the 10-day full charge storage test and 30-day full charge storage test.

In Comparative Examples 34 to 36 which used nonaqueous electrolyte solutions that did not contain acetonitrile, the residual capacity and recovery capacity retention rate after 10 days were excellent, but the post-recovery efficiency after 10 days was low and irreversible degradation reaction had progressed. In Comparative Example 36 as well, which was acceptable after 10 days, the residual capacity, post-recovery efficiency and recovery capacity retention rate after 30 days were lowered, confirming inferior high temperature durability. For this embodiment, appropriate adjustment of the amount of $LiPF_6$ can not only inhibit "removal of hydrogen from the $\alpha$-position of acetonitrile and generation of excess HF from $PF_6$ anions", but since $LiPF_6$ thermal decomposition products are also reduced, it can also inhibit continuous degradation reaction by a carbonate electrolyte solution.

When Example 22 and Comparative Example 33 were compared, it was confirmed that the residual capacity retention rate, post-recovery charge-discharge efficiency and recovery capacity retention rate were improved in Example 22, despite both having the same nonaqueous solvent composition. This suggests that lowering the amount of $LiPF_6$ to appropriately adjust the mixing ratio of $LiPF_6$ and lithium-containing imide salt can inhibit degradation at high temperature and can drastically improve the prolonged storage property. Comparative Example 33 exhibited abnormal voltage after storage for 30 days, and this is attributed to elution of metal from the positive electrode by HF generated during the storage period, leading to precipitation at the negative electrode end and consequent short circuiting.

These results demonstrate that the nonaqueous secondary battery of this embodiment can exhibit improved prolonged storage properties at 85°C without having impaired output characteristics in low temperature environments, even when using an electrolyte solution containing a relatively large amount of acetonitrile in the nonaqueous solvent.

<Third embodiment>

(7-1) Assembly of coin-type nonaqueous secondary battery (P1/N1)

A polypropylene gasket was set in a CR2032 type battery case (SUS304/A1 cladding), and a positive electrode (P1) obtained as described above, punched into a discoid shape with a diameter of 15.958 mm, was set at the center with the positive electrode active material layer facing upward. Glass fiber filter paper (GA-100, product of Advantech, Inc.) punched out into a discoid shape with a diameter of 16.156 mm was then set over it, and 150 $\mu$L of a nonaqueous electrolyte solution (S14 to S22, S24, S25, S32, S36, S44 and S45; none of which are in accordance with the claimed invention) was injected in, after which a negative electrode (N1) obtained as described above, punched into a discoid shape with a diameter of 16.156 mm, was set with the negative electrode active material layer facing downward. After further setting a spacer and spring inside the battery case, a battery cap was fitted over and a caulking seal was formed with a caulking machine. The overflowing electrolyte solution was wiped off with a waste cloth. The battery was stored at 25 °C for 12 hours to allow sufficient interaction of the nonaqueous electrolyte solution with the layered product, to obtain a coin-type nonaqueous secondary battery (P1/N1).

(7-2) Initial charge-discharge processing for coin-type nonaqueous secondary battery (P1/N1)

The coin-type nonaqueous secondary battery (P1/N1) obtained in (7-1) was subjected to initial charge-discharge processing by the same procedure as (4-1), and the initial efficiency was calculated.

(7-3) Full charge storage test at 85°C for coin-type nonaqueous secondary battery (P1/N1)

The coin-type nonaqueous secondary battery (P1/N1) that had been subjected to initial charge-discharge processing by the method described in (7-2) above, was subjected to a full charge storage test at 85°C by the method described in (4-2-1) above, and this was followed by battery performance calculation by the methods described in (4-3) above. Interpretation of the test results is as explained in (4-3-1) above.

(7-4) 200-cycle operation test at 25°C for coin-type nonaqueous secondary battery (P1/N1)

The coin-type nonaqueous secondary battery (P1/N1) which had been subjected to accelerated aging by the method described in (7-3) above was subjected to a cycle test. The cycle test was carried out with the ambient temperature of the battery set to 25°C. First, charge was carried out at a constant current of 6.9 mA corresponding to 1.5 C to reach 3.8 V, and then charge was carried out at a constant voltage of 3.8 V, until the current was attenuated to 0.23 mA corresponding to 0.05 C. The battery was then discharged to 2.5 V at a constant current of 6.9 mA. With the step of a single charge and discharge defined as one cycle, charge-discharge was carried out for 200 cycles. The discharge

capacity at the 200th cycle, assuming the discharge capacity in the first cycle to be 100%, was recorded as the capacity retention, with an acceptable level considered to be a capacity retention of >70% after 200 cycles. The evaluation results are shown in Table 14.

[Table 14]

| | Electrolyte solution No. | $LiPF_6$ content (mol/1 L solvent) | Imide salt | Imide salt content (mol/1 L solvent) | Molar ratio of $LiPF_6$ and imide salt | First initial charge-discharge efficiency [%] | 0.3 C Residual capacity retention rate [%] | Charge-discharge efficiency [%] | 0.3 C Recovery capacity retention rate [%] | 1.5 C Recovery capacity retention rate [%] | Operating test after 200 cycles |
|---|---|---|---|---|---|---|---|---|---|---|---|
| Example 23 | S15 | 0.075 | LiFSI | 1.225 | 16.3 | 89.7 | 92.6 | 99.6 | 91.9 | 92.0 | Acceptable |
| Example 24 | S16 | 0.050 | LiFSI | 1.250 | 25.0 | 89.8 | 92.4 | 99.9 | 92.1 | 92.3 | Acceptable |
| Example 25 | S17 | 0.025 | LiFSI | 1.275 | 51.0 | 90.0 | 92.2 | 99.9 | 91.9 | 92.1 | Acceptable |
| Example 26 | S18 | 0.050 | LiFSI | 1.250 | 25.0 | 90.6 | 92.7 | 99.5 | 91.5 | 91.7 | Acceptable |
| Example 27 | S19 | 0.025 | LiFSI | 1.275 | 51.0 | 90.8 | 92.6 | 99.2 | 92.2 | 90.8 | Acceptable |
| Example 28 | S20 | 0.075 | LiFSI | 1.225 | 16.3 | 91.0 | 92.6 | 99.3 | 91.4 | 91.5 | Acceptable |
| Example 29 | S21 | 0.050 | LiFSI | 1.250 | 25.0 | 91.3 | 94.6 | 99.9 | 95.8 | 95.1 | Acceptable |
| Example 30 | S22 | 0.050 | LiFSI | 1.250 | 25.0 | 92.0 | 94.5 | 99.9 | 95.3 | 94.2 | Acceptable |
| Example 31 | S14 | 0.050 | LiFSI | 1.250 | 25.0 | 90.5 | 93.9 | 99.8 | 94.7 | 93.6 | Acceptable |
| Comparative Example 37 | S44 | - | LiFSI | 1.300 | - | 89.8 | 91.3 | 98.9 | 92.1 | 91.1 | Abnormal capacity |
| Comparative Example 38 | S32 | 0.300 | LiFSI | 1.000 | 3.3 | 90.0 | 90.9 | 98.4 | 91.0 | 88.6 | Acceptable |
| Comparative Example 39 | S45 | - | LiFSI | 1.300 | - | 89.9 | 91.0 | 99.1 | 91.5 | 90.3 | Abnormal capacity |
| Comparative Example 40 | S36 | 0.300 | LiFSI | 1.000 | 3.3 | 91.7 | 89.7 | 97.3 | 89.9 | 85.9 | Acceptable |
| Comparative Example 41 | S24 | 1.000 | - | - | - | 91.5 | 91.4 | 99.7 | 94.2 | 90.7 | Acceptable |
| Comparative Example 42 | S25 | 1.200 | - | - | - | 92.1 | 86.3 | 99.4 | 92.2 | 89.4 | Acceptable |

**Examples 23 to 31 are not in accordance with** the **invention as claimed.**

When Examples 23 to 25 **(not according to the claimed invention)** and Comparative Example 38 were compared, the residual capacity retention rate, charge-discharge efficiency and recovery capacity retention rate were found to be improved in Examples 23 to 25, despite all of them having the same nonaqueous solvent composition.

Comparing Examples 23 to 25 **(not according to the claimed invention)** and Comparative Example 37, the battery failed to operate up to 200 cycles in Comparative Example 37 and malfunctioned due to abnormal capacity during the course of cycle testing, despite all of them having the same nonaqueous solvent composition.

Comparing Examples 26 to 28 **(not according to the claimed invention)** and Comparative Example 39, the battery failed to operate up to 200 cycles in Comparative Example 39 and malfunctioned due to abnormal capacity during the course of cycle testing, despite all of them having the same nonaqueous solvent composition.

When Examples 26 to 28 and Comparative Example 40 were compared, it was found that the residual capacity retention rate, charge-discharge efficiency and recovery capacity retention rate were improved in Examples 26 to 28, despite all of them having the same nonaqueous solvent composition.

Examples 29 to 30 **(not according to the claimed invention)** likewise had improved residual capacity retention rate, charge-discharge efficiency and recovery capacity retention rate compared to Comparative Examples 37 to 40.

Comparative Examples 37 and 39 with abnormal capacity did not contain $LiPF_6$, and exhibited progressive corrosion of the Al current collector or had reductive decomposition of the electrolyte solution component due to insufficient negative electrode SEI formation, resulting in shutdown presumably due to abnormal capacity.

With Comparative Examples 41 and 42, the 1.5 C recovery capacity retention rate was significantly reduced. It is thought that addition of a large amount of $LiPF_6$ increased the internal resistance during the high temperature storage test, thereby lowering the output characteristic.

These results confirmed that using a nonaqueous electrolyte solution according to this embodiment can minimize corrosion of the positive electrode or current collector, or reduction in battery performance due to degradation reaction of the electrolyte solution, and can thus improve battery performance.

It was demonstrated by these results that the nonaqueous secondary battery of this embodiment can improve battery performance even with a nonaqueous secondary battery using an electrolyte solution based on acetonitrile.

## INDUSTRIAL APPLICABILITY

The nonaqueous electrolyte solution and nonaqueous secondary battery of the invention is expected to be useful in battery chargers for portable devices such as cellular phones, portable audio devices, personal computers and IC (Integrated Circuit) tags; battery chargers for automobiles such as hybrid vehicles, plug-in hybrid vehicles and electric vehicles; low voltage power sources such as 12 V class power sources, 24 V class power sources and 48 V class power sources; and home power storage systems or IoT appliances. The nonaqueous secondary battery of the invention can also be applied for cold climate purposes, and summer season outdoor purposes.

## REFERENCE SIGNS LIST

- 100 Nonaqueous secondary battery
- 110 Battery exterior
- 120 Battery exterior space
- 130 Positive electrode lead
- 140 Negative electrode lead
- 150 Positive electrode
- 160 Negative electrode
- 170 Separator

## Claims

**1.** A nonaqueous electrolyte solution comprising:

a nonaqueous solvent containing acetonitrile at 5 vol% to 95 vol%;
a lithium salt; and
one or more compounds having a structure satisfying the following conditions 1 to 5:

1. being a condensation polycyclic heterocyclic ring compound,
2. containing a pyrimidine backbone in the condensation polycyclic heterocyclic ring,
3. containing 3 or more nitrogen atoms in the condensation polycyclic heterocyclic ring,
4. containing 5 or more sp2 carbons in the condensation polycyclic heterocyclic ring, and
5. having no hydrogen atoms bonded to the nitrogen atoms in the condensation polycyclic heterocyclic ring.

**2.** The nonaqueous electrolyte solution according to claim 1, wherein the condensation polycyclic heterocyclic ring compound is at least one selected from the group consisting of compounds represented by the following formulas (1) to (12):

[Chemical Formula 1]

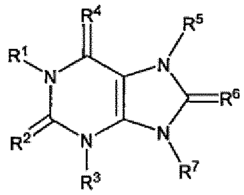
(1)
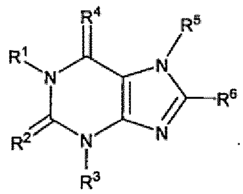
(2)
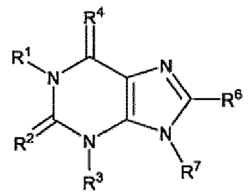
(3)
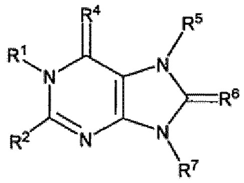
(4)
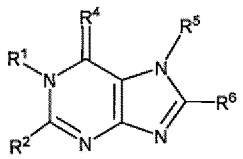
(5)
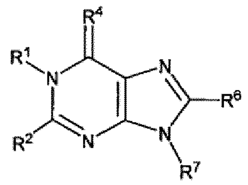
(6)
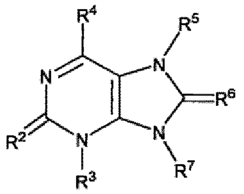
(7)
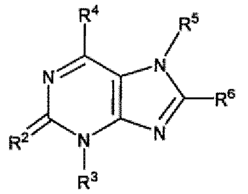
(8)
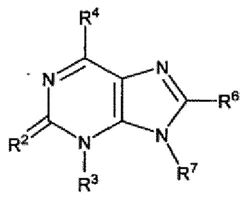
(9)
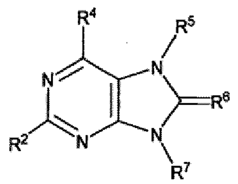
(10)
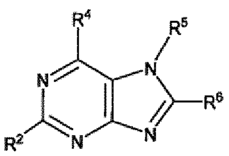
(11)
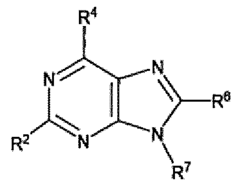
(12)

where $R^2$, $R^4$ and $R^6$ which form double bonds with carbon atoms in the condensation polycyclic heterocyclic ring represent oxygen atoms or sulfur atoms, and $R^2$, $R^4$ and $R^6$ which form single bonds with carbon atoms in the condensation polycyclic heterocyclic ring and $R^1$, $R^3$, $R^5$ and $R^7$ which bond with nitrogen atoms in the condensation polycyclic heterocyclic ring represent alkyl groups of 1 to 4 carbon atoms, haloalkyl groups of 1 to 4 carbon atoms, acylalkyl groups of 1 to 4 carbon atoms, allyl, propargyl, phenyl, benzyl, pyridyl, amino, pyrrolidylmethyl, trimethylsilyl, acetyl, trifluoroacetyl, chloromethyl, methoxymethyl, isocyanomethyl, methylsulfonyl, 2-(trimethylsilyl)-ethoxycarbonyloxy, bis(N,N'-alkyl)aminomethyl or bis(N,N'-alkyl)aminoethyl groups, alkoxy groups of 1 to 4 carbon atoms, fluorine-substituted alkoxy groups of 1 to 4 carbon atoms, nitrile groups, nitro groups, halogen atoms, and saccharide residues or heterocyclic ring residues; with the proviso that $R^2$, $R^4$ and $R^6$ that form single bonds with carbon atoms in the condensation polycyclic heterocyclic ring are optionally hydrogen atoms, and their isomers.

**3.** The nonaqueous electrolyte solution according to claim 2, wherein the condensation polycyclic heterocyclic ring compound is at least one selected from the group consisting of compounds represented by formulas (2), (5), (8) and (12), and their isomers.

**4.** The nonaqueous electrolyte solution according to claim 3, wherein the condensation polycyclic heterocyclic ring compound is a compound represented by formula (2), or an isomer thereof.

5. The nonaqueous electrolyte solution according to any one of claims 1 to 4, wherein the condensation polycyclic heterocyclic ring compound is caffeine.

6. The nonaqueous electrolyte solution according to any one of claims 1 to 5, wherein the content of the condensation polycyclic heterocyclic ring compound is 0.01 weight% to 10 weight% based on the total weight of the nonaqueous electrolyte solution.

7. The nonaqueous electrolyte solution according to any one of claims 1 to 6, wherein the lithium salt includes $LiPF_6$ and a lithium-containing imide salt.

8. The nonaqueous electrolyte solution according to claim 7, wherein the content of the $LiPF_6$ is 0.01 mol/L or greater and less than 0.1 mol/L with respect to the nonaqueous solvent.

9. The nonaqueous electrolyte solution according to claim 7 or 8, wherein the molar ratio of the lithium-containing imide salt with respect to the $LiPF_6$ is greater than 10.

10. The nonaqueous electrolyte solution according to any one of claims 7 to 9, wherein the lithium-containing imide salt includes lithium bis(fluorosulfonyl)imide.

11. A nonaqueous secondary battery including the nonaqueous electrolyte solution according to any one of claims 1 to 10.

12. The nonaqueous secondary battery according to claim 11, comprising:

a positive electrode that contains a lithium-phosphorus metal compound with an Fe-containing olivine crystal structure, and
a negative electrode that contains graphite or one or more elements selected from the group consisting of Ti, V, Sn, Cr, Mn, Fe, Co, Ni, Zn, Al, Si and B.

13. The nonaqueous secondary battery according to claim 12, wherein the basis weight of the positive electrode per side is 15 $mg/cm^2$ or greater.

**Patentansprüche**

1. Nichtwässrige Elektrolytlösung, umfassend:

ein nichtwässriges Lösungsmittel, das Acetonitril in einem Anteil von 5 Vol.-% bis 95 Vol.-% enthält;
ein Lithiumsalz; und
eine oder mehrere Verbindungen mit einer Struktur, die den folgenden Bedingungen 1 bis 5 genügt:

1. es handelt sich um eine ringkondensierte polycyclische heterocyclische Verbindung;
2. sie enthält ein Pyrimidingerüst in dem ringkondensierten polycyclischen Heterocyclus;
3. sie enthält 3 oder mehr Stickstoffatome in dem ringkondensierten polycyclischen Heterocyclus;
4. sie enthält 5 oder mehr sp2-Kohlenstoffatome in dem ringkondensierten polycyclischen Heterocyclus; und
5. sie weist keine Wasserstoffatome auf, die an die Stickstoffatome in dem ringkondensierten polycyclischen Heterocyclus gebunden sind.

2. Nichtwässrige Elektrolytlösung gemäß Anspruch 1, wobei die ringkondensierte polycyclische heterocyclische Verbindung wenigstens eine ist, die aus der Gruppe ausgewählt ist, die aus Verbindungen besteht, die durch die folgenden Formeln (1) bis (12) dargestellt werden:

[chemische Formel 1]

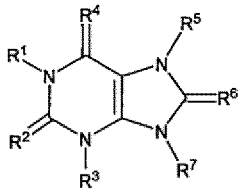 (1)

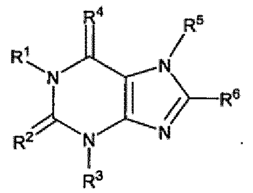 (2)

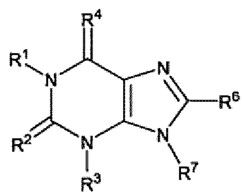 (3)

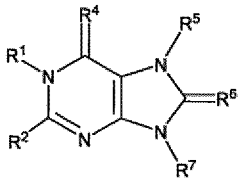 (4)

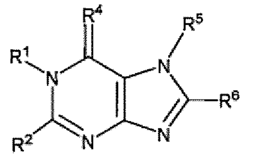 (5)

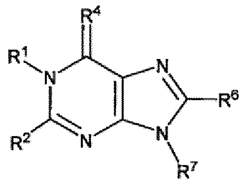 (6)

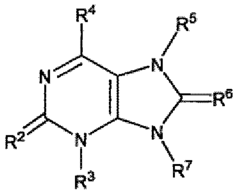 (7)

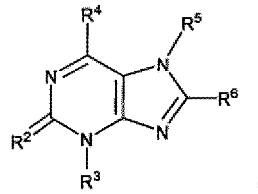 (8)

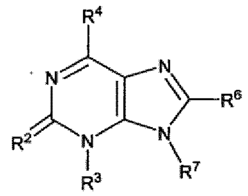 (9)

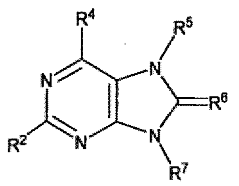 (10)

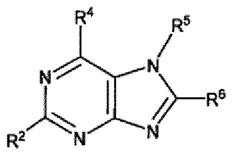 (11)

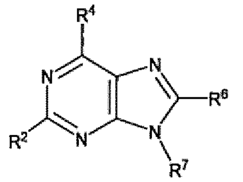 (12)

wobei $R^2$, $R^4$ und $R^6$, die Doppelbindungen mit Kohlenstoffatomen in dem ringkondensierten polycyclischen Heterocyclus bilden, für Sauerstoffatome oder Schwefelatome stehen und $R^2$, $R^4$ und $R^6$, die Einfachbindungen mit Kohlenstoffatomen in dem ringkondensierten polycyclischen Heterocyclus bilden, und $R^1$, $R^3$, $R^5$ und $R^7$, die Bindungen mit Stickstoffatomen in dem ringkondensierten polycyclischen Heterocyclus bilden, für Alkylgruppen mit 1 bis 4 Kohlenstoffatomen, Halogenalkylgruppen mit 1 bis 4 Kohlenstoffatomen, Acylalkylgruppen mit 1 bis 4 Kohlenstoffatomen, Allyl-, Propargyl- , Phenyl-, Benzyl-, Pyridyl-, Amino-, Pyrrolidylmethyl-, Trimethylsilyl-, Acetyl-, Trifluoracetyl-, Chlormethyl-, Methoxymethyl-, Isocyanomethyl-, Methylsulfonyl-, 2-(Trimethylsilyl)ethoxycarbonyloxy-, Bis(N,N'-alkyl)-aminomethyl- oder Bis(N,N'-alkyl)aminoethyl-Gruppen, Alkoxygruppen mit 1 bis 4 Kohlenstoffatomen, fluorsubstituierten Alkoxygruppen mit 1 bis 4 Kohlenstoffatomen, Nitrilgruppen, Nitrogruppen, Halogenatomen und Saccharid-Resten oder Resten heterocyclischer Ringe stehen; mit der Maßgabe, dass $R^2$, $R^4$ und $R^6$, die Einfachbindungen mit Kohlenstoffatomen in dem ringkondensierten polycyclischen Heterocyclus bilden, gegebenenfalls Wasserstoffatome sind,
und deren Isomere.

**3.** Nichtwässrige Elektrolytlösung gemäß Anspruch 2, wobei die ringkondensierte polycyclische heterocyclische Verbindung wenigstens eine ist, die aus der Gruppe ausgewählt ist, die aus Verbindungen, welche durch die Formeln (2), (5), (8) und (12) dargestellt werden, und deren Isomeren besteht.

**4.** Nichtwässrige Elektrolytlösung gemäß Anspruch 3, wobei die ringkondensierte polycyclische heterocyclische Verbindung eine durch Formel (2) dargestellte Verbindung oder ein Isomer davon ist.

**5.** Nichtwässrige Elektrolytlösung gemäß einem der Ansprüche 1 bis 4, wobei es sich bei der ringkondensierten polycyclischen heterocyclischen Verbindung um Coffein handelt.

**6.** Nichtwässrige Elektrolytlösung gemäß einem der Ansprüche 1 bis 5, wobei der Gehalt an der ringkondensierten polycyclischen heterocyclischen Verbindung 0,01 Gew.-% bis 10 Gew.-% beträgt, bezogen auf das Gesamtgewicht der nichtwässrigen Elektrolytlösung.

7. Nichtwässrige Elektrolytlösung gemäß einem der Ansprüche 1 bis 6, wobei das Lithiumsalz $LiPF_6$ und ein lithiumhaltiges Imidsalz umfasst.

8. Nichtwässrige Elektrolytlösung gemäß Anspruch 7, wobei der Gehalt an dem $LiPF_6$ 0,01 mol/l oder mehr und weniger als 0,1 mol/l in Bezug auf das nichtwässrige Lösungsmittel beträgt.

9. Nichtwässrige Elektrolytlösung gemäß Anspruch 7 oder 8, wobei das Stoffmengenverhältnis des lithiumhaltigen Imidsalzes zu dem $LiPF_6$ größer als 10 ist.

10. Nichtwässrige Elektrolytlösung gemäß einem der Ansprüche 7 bis 9, wobei das lithiumhaltige Imidsalz Lithiumbis(fluorsulfonyl)imid umfasst.

11. Nichtwässrige Sekundärbatterie, die die nichtwässrige Elektrolytlösung gemäß einem der Ansprüche 1 bis 10 umfasst.

12. Nichtwässrige Sekundärbatterie gemäß Anspruch 11, umfassend:

eine positive Elektrode, die eine Lithium-Phosphor-Metallverbindung mit einer Fe-haltigen Olivin-Kristallstruktur enthält, und
eine negative Elektrode, die Graphit oder ein oder mehrere Elemente enthält, die aus der Gruppe ausgewählt sind, die aus Ti, V, Sn, Cr, Mn, Fe, Co, Ni, Zn, Al, Si und B besteht.

13. Nichtwässrige Sekundärbatterie gemäß Anspruch 12, wobei das Flächengewicht der positiven Elektrode pro Seite 15 $mg/cm^2$ oder mehr beträgt.

## Revendications

1. Solution d'électrolyte non aqueuse comprenant :

un solvant non aqueux contenant de l'acétonitrile à de 5 % en volume à 95 % en volume ;
un sel de lithium ; et
un ou plusieurs composés ayant une structure satisfaisant les conditions 1 à 5 suivantes :

1. être un composé à noyau hétérocyclique polycyclique de condensation,
2. contenir un squelette de pyrimidine dans le noyau hétérocyclique polycyclique de condensation,
3. contenir 3 atomes d'azote ou plus dans le noyau hétérocyclique polycyclique de condensation,
4. contenir 5 carbones sp2 ou plus dans le noyau hétérocyclique polycyclique de condensation, et
5. n'avoir aucun atome d'hydrogène lié aux atomes d'azote dans le noyau hétérocyclique polycyclique de condensation.

2. Solution d'électrolyte non aqueuse selon la revendication 1, dans laquelle le composé de noyau hétérocyclique polycyclique de condensation est au moins un choisi dans le groupe consistant en composés représentés par les formules (1) à (12) suivantes :

[Formule chimique 1]

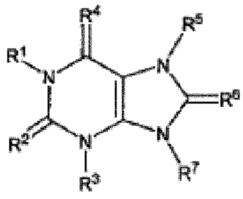 (1) 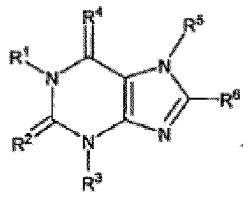 (2) 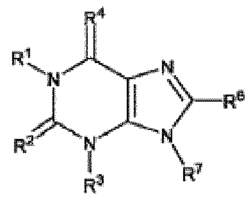 (3)

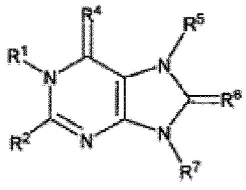 (4) 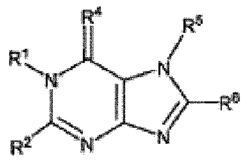 (5) 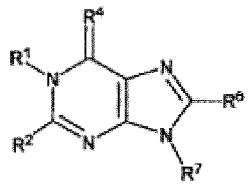 (6)

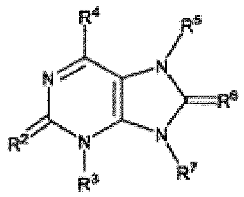 (7) 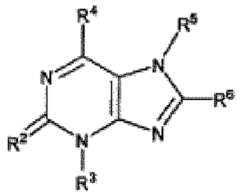 (8) 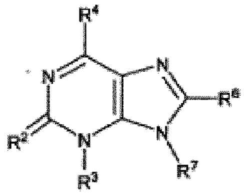 (9)

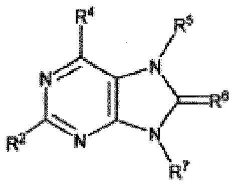 (10) 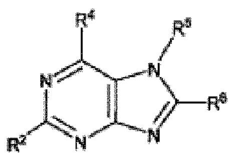 (11) 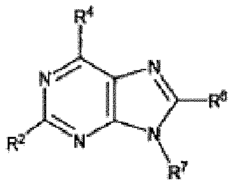 (12)

où
$R^2$, $R^4$ et $R^6$ qui forment des doubles liaisons avec des atomes de carbone dans le noyau hétérocyclique polycyclique de condensation représentent des atomes d'oxygène ou atomes de soufre, et $R^2$, $R^4$ et $R^5$ qui forment des liaisons simples avec des atomes de carbone dans le noyau hétérocyclique polycyclique de condensation et $R^1$, $R^3$, $R^5$ et $R^7$ qui sont liés avec des atomes d'azote dans le noyau hétérocyclique polycyclique de condensation représentent des groupes alkyle de 1 à 4 atomes de carbone, groupes haloalkyle de 1 à 4 atomes de carbone, groupes acylalkyle de 1 à 4 atomes de carbone, groupes allyle, propargyle, phényle, benzyle, pyridyle, amino, pyrrolidylméthyle, triméthylsilyle, acétyle, trifluoroacétyle, chlorométhyle, méthoxyméthyle, isocyanométhyle, méthylsulfonyle, 2-(triméthylsilyl)-éthoxycarbonyloxy, bis(N,N'-alkyl)aminométhyle ou bis(N,N'-alkyl)aminoéthyle, groupes alcoxy de 1 à 4 atomes de carbone, groupes alcoxy substitués par du fluor de 1 à 4 atomes de carbone, groupes nitrile, groupes nitro, atomes d'halogène, et résidus de saccharides ou résidus de noyaux hétérocycliques ; à condition que $R^2$, $R^4$ et $R^6$ qui forment des liaisons simples avec des atomes de carbone dans le noyau hétérocyclique polycyclique de condensation sont éventuellement des atomes d'hydrogène,
et leurs isomères.

**3.** Solution d'électrolyte non aqueuse selon la revendication 2, dans laquelle le composé de noyau hétérocyclique polycyclique de condensation est au moins un choisi dans le groupe consistant en composés représentés par les formules (2), (5), (8) et (12), et leurs isomères.

**4.** Solution d'électrolyte non aqueuse selon la revendication 3, dans laquelle le composé de noyau hétérocyclique polycyclique de condensation est un composé représenté par la formule (2), ou un isomère de celui-ci.

**5.** Solution d'électrolyte non aqueuse selon l'une quelconque des revendications 1 à 4, dans laquelle le composé de noyau hétérocyclique polycyclique de condensation est la caféine.

**6.** Solution d'électrolyte non aqueuse selon l'une quelconque des revendications 1 à 5, dans laquelle la teneur du composé de noyau hétérocyclique polycyclique de condensation est de 0,01 % en masse à 10 % en masse sur la base de la masse totale de la solution d'électrolyte non aqueuse.

**7.** Solution d'électrolyte non aqueuse selon l'une quelconque des revendications 1 à 6, dans laquelle le sel de lithium inclut $LiPF_6$ et un sel d'imide contenant du lithium.

**8.** Solution d'électrolyte non aqueuse selon la revendication 7, dans laquelle la teneur du $LiPF_6$ est de 0,01 mol/L ou supérieure et inférieure à 0,1 mol/L par rapport au solvant non aqueux.

**9.** Solution d'électrolyte non aqueuse selon la revendication 7 ou 8, dans laquelle le rapport molaire du sel d'imide contenant du lithium par rapport au $LiPF_6$ est supérieur à 10.

**10.** Solution d'électrolyte non aqueuse selon l'une quelconque des revendications 7 à 9, dans laquelle le sel d'imide contenant du lithium inclut du lithium bis(fluorosulfonyl)imide.

**11.** Accumulateur secondaire non aqueux incluant la solution d'électrolyte non aqueuse selon l'une quelconque des revendications 1 à 10.

**12.** Accumulateur secondaire non aqueux selon la revendication 11, comprenant :

une électrode positive qui contient un composé de métal de lithium-phosphore avec une structure de cristal d'olivine contenant Fe, et
une électrode négative qui contient du graphite ou un ou plusieurs éléments choisis dans le groupe consistant en Ti, V, Sn, Cr, Mn, Fe, Co, Ni, Zn, Al, Si et B.

**13.** Accumulateur secondaire non aqueux selon la revendication 12, dans lequel la masse de base de l'électrode positive par côté est de 15 $mg/cm^2$ ou supérieure.

FIG. 1

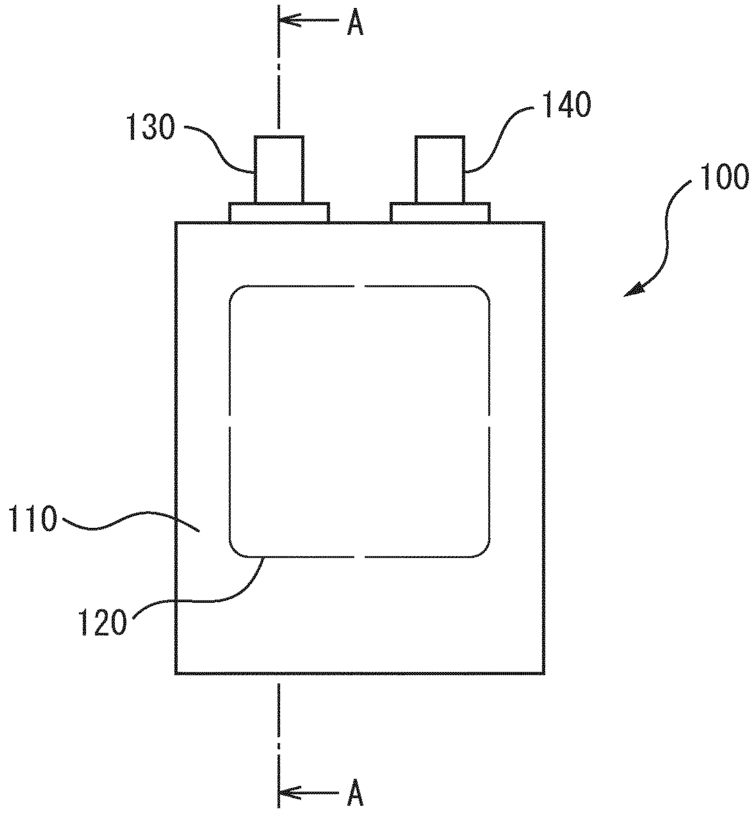


FIG. 2

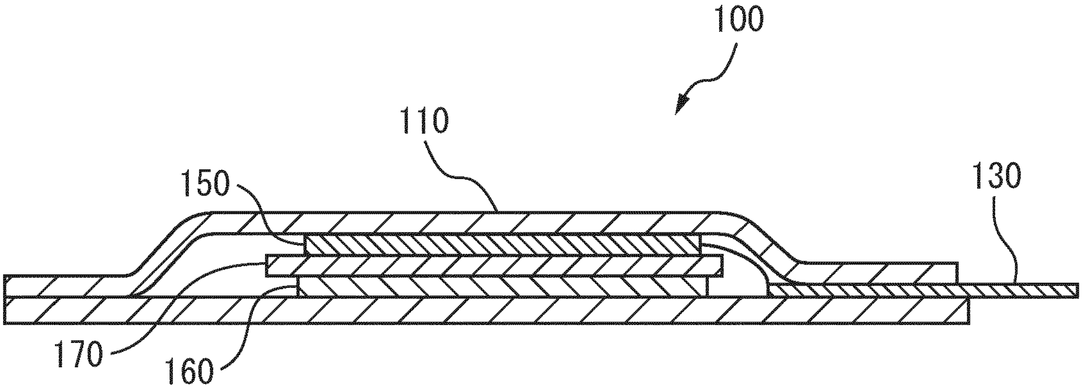

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001148257 A
- US 2012251892 A1
- WO 2013062056 A
- JP H041992351860 A
- JP 2009021134 A
- WO 2013146714 A
- WO 2016159117 A
- WO 2013183673 A
- WO 2016068022 A
- JP 2001307737 A
- US 20120251892